# FASCICULE DE BREVET EUROPEEN

(11) **EP 1 018 508 B1**
(45) Date de publication et mention de la délivrance du brevet: **04.05.2005**
(21) Numéro de dépôt: 99403168.0
(22) Date de dépôt: 16.12.1999
(51) Int. Cl.: C07D 233/61, A61K 7/13

(54) **Nitrophenylenediamines cationiques monobenzéniques, leur utilisation pour la teinture des fibres kératiniques, compositions tinctoriales les renfermant et procédés de teinture**
Kationische Monobenzol-nitrophenylendiamine, ihre Verwendung zum Färben von Keratinfasern, sie enthaltende Färbemittelzusammensetzungen und Färbeverfahren
Cationic monobenzenic nitrophenylene diamines, their use for dyeing keratinic fibres, dye compositions containing them and dye processes

(30) Priorité: 08.01.1999 FR 9900150
(43) Date de publication de la demande: 12.07.2000
(73) Titulaire: L'OREAL, 75008 Paris (FR)
(72) Inventeur: Genet, Alain, 93600 Aulnay Sous Bois (FR); Lagrange, Alain, 77700 Coupvray (FR)
(74) Mandataire: Miszputen, Laurent

(56) Documents cités:
- EP-A- 0 673 926
- FR-A- 2 520 358
- FR-A- 2 766 178
- GB-A- 909 700
- GB-A- 1 164 824
- US-A- 4 018 556
- US-A- 5 139 532

## Description

La présente invention concerne des nitrophénylènediamines monobenzéniques comportant au moins un groupement cationique choisi parmi des chaînes aliphatiques comportant au moins une charge cationique délocalisée sur un hétérocycle polyazoté insaturé à 5 chaînons, leur utilisation à titre de colorant direct dans les applications de teinture des matières kératiniques, en particulier des fibres kératiniques humaines, et notamment les cheveux, et plus particulièrement les compositions de teinture les renfermant.

Il est connu de teindre les fibres kératiniques, et en particulier les cheveux, avec des compositions tinctoriales contenant des colorants directs, c'est-à-dire des molécules colorantes ayant une affinité pour lesdites fibres. Le procédé de teinture qui les met en oeuvre est un procédé dit de coloration directe qui consiste à laisser pauser les colorants directs sur les fibres, puis à les rincer.
Les colorations qui en résultent sont des colorations temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Parmi les colorants directs connus, on a certes déjà décrit des nitrophénylènediamines cationiques mais leur charge cationique est localisée sur l'atome d'azote d'une chaîne aliphatique ou d'un hétérocycle à 6 chaînons, saturé et monoazoté comme la morpholine ou la pipéridine.
De telles nitrophénylènediamines sont par exemple décrites dans les brevets anglais N°- 1 164 824, américain N°- 4 018 556 ou EP-A 0 673 926.

Cependant, en teinture capillaire, on recherche en permanence des colorants directs qui présentent des caractéristiques toujours plus performantes.

C'est donc après d'importantes recherches menées sur la question que la demanderesse vient maintenant de découvrir de façon tout à fait inattendue et surprenante, de nouvelles nitrophénylènediamines cationiques monobenzéniques, dont au moins une charge cationique est délocalisée sur un hétérocycle polyazoté insaturé à 5 chaînons et comportant donc au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé.
Cette nouvelle famille de colorants présente la particularité très avantageuse d'une plus grande solubilité dans les milieux de teinture, et ces nouveaux colorants engendrent des teintures, par coloration directe, dotées d'une puissance et d'une résistance (aux diverses agressions que peuvent subir les cheveux : lumière, frottement, intempéries, shampooings, transpiration) notablement améliorée, par rapport à celles des teintures réalisées avec des nitrophénylènediamines cationiques connues de l'art antérieur dont la charge cationique est localisée sur l'atome d'azote d'une chaîne aliphatique ou d'un hétérocycle monoazoté.

Cette découverte est à la base de la présente invention.

La présente invention a ainsi pour objet les nitrophénylènediamines cationiques monobenzéniques de formule (I) suivante : 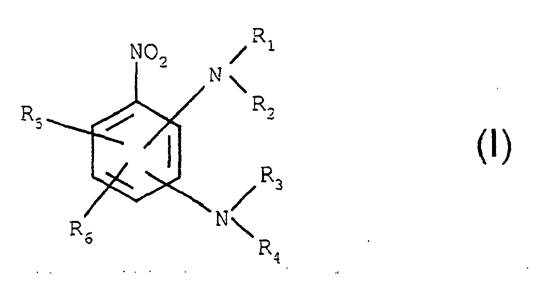 formule dans laquelle,
- **R**_{**1**}, **R**_{**2**}, **R**_{**3**}, et **R**_{**4**}, qui peuvent être identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle.
- **R**_{**5**}, **R**_{**6**}, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical aminoalkyle(C₁-C₆) dont l'alkyle est substitué ou non substitué par un ou plusieurs radicaux hydroxy et dont l'amine est substituée ou non substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle; un groupement OR₇ ou -SR₇ défini ci-après.
- **R**_{**7**} désigne un atome d'hydrogène ; un radical alkyle en C₁-C₆; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle.
- **Z** est choisi parmi les groupements cationiques insaturés de formules (II) suivant et les groupements cationiques saturés de formule (III) suivante : 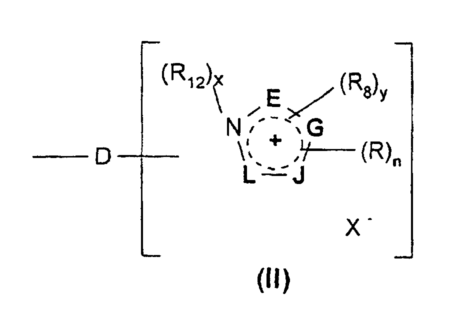 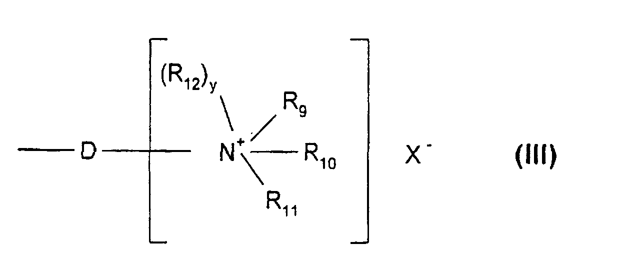
dans lesquelles :
- D est un bras de liaison qui représente une chaîne alkyle comportant de préférence de 1 à 14 atomes de carbone, linéaire ou ramifiée pouvant être substituée par un ou plusieurs radicaux hydroxyle ou alcoxy en C₁-C₆.
- les sommets E, G, J et L, identiques ou différents, représentent un atome de carbone ou d'azote ;
- n est un nombre entier compris entre 0 et 4 inclusivement;
- les radicaux R, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₈ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle.
- R₉, R₁₀ et R₁₁, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₉, R₁₀ et R₁₁ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.
- R₁₂ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x et y sont des nombres entiers égaux à 0 ou 1 ; avec les conditions suivantes :
   - dans les groupements cationiques insaturés de formule (II) :
      - lorsque x = 0, le bras de liaison D est rattaché à l'atome d'azote,
      - lorsque x = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₈ est porté par l'atome d'azote du cycle insaturé ; ou bien
         2) lorsqu'au moins un des sommets E, G, J et L représente un atome d'azote sur lequel le radical R₈ est fixé ;
   - dans les groupements cationiques de formule (III) :
      - lorsque y = 0, alors le bras de liaison D est rattaché à l'atome d'azote portant les radicaux R₉ à R₁₁,
      - lorsque y = 1, alors deux des radicaux R₉ à R₁₁ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
- **X**⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu :**
- que le nombre de groupements cationiques insaturés Z de formule (II), dans lesquels au moins un des sommets E, G, J et L représente un atome d'azote, est au moins égal à 1.

Les alkyles et alkoxy cités ci-avant dans les formules (I), (II) et (III) peuvent être linéaires ou ramifiés.

Les composés de formule (I) peuvent être éventuellement salifiés par des acides minéraux forts tels que HCI, HBr, H₂SO₄, ou des acides organiques tels qu'acétique, tartrique, lactique, citrique ou succinique.

Parmi les cycles des groupements insaturés Z de formule (II) ci-dessus, on peut notamment citer à titre d'exemple les cycles pyrrolique, imidazolique, pyrazolique, et triazolique.

Les composés de formule (I) sont de préférence choisis parmi ceux de formules (I)₁ à (I)₁₅ suivantes : 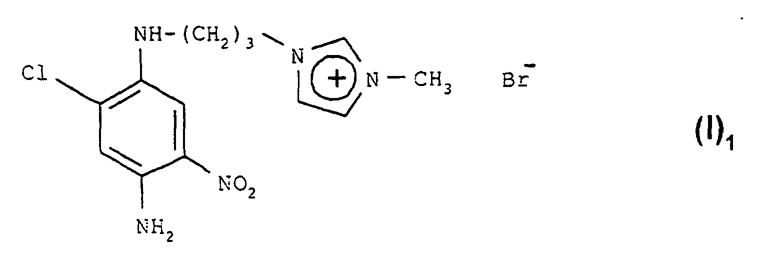 i.e. bromure du 1-[3-(4-amino-2-chloro-5-nitro-phénylamino)-propyl]-3-méthyl-3H-imidazol-1-ium, 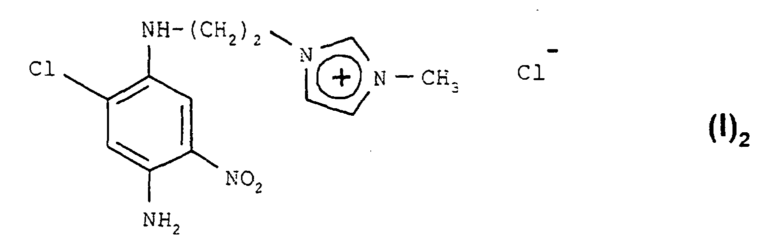 i.e. chlorure du 1-[2-(4-amino-2-chloro-5-nitro-phénylamino)-éthyl]-3-méthyl-3H-imidazol-1-ium, 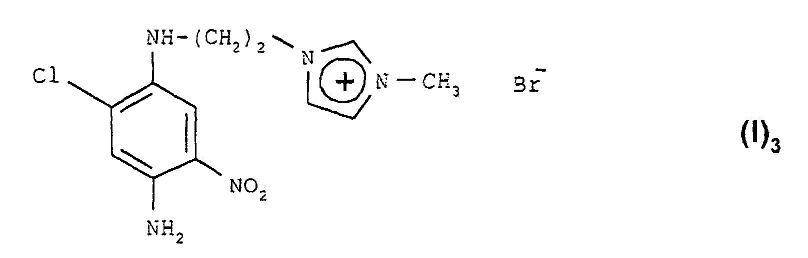 i.e. bromure du 1-[2-(4-amino-2-chloro-5-nitro-phénylamino)-éthyl]-3-méthyl-3H-imidazol-1-ium, 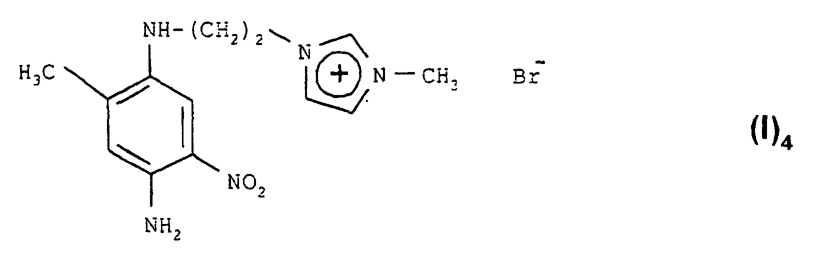 i.e. bromure du 1-[2-(4-amino-2-méthyl-5-nitro-phénylamino)-éthyl]-3-méthyl-3H-imidazol-1-ium, 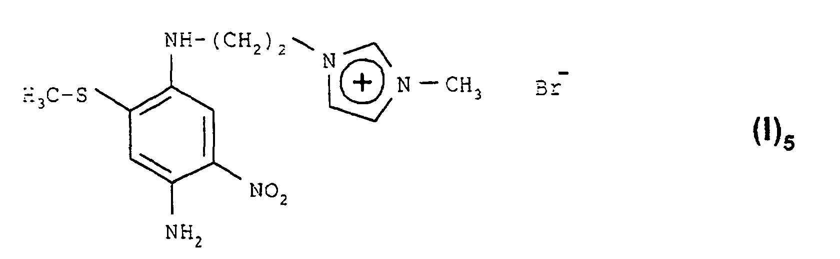 i.e. bromure du 1-[2-(4-amino-2-méthylsulfanyl-5-nitro-phénylamino)-éthyl]-3-méthyl-3H-imidazol-1-ium, 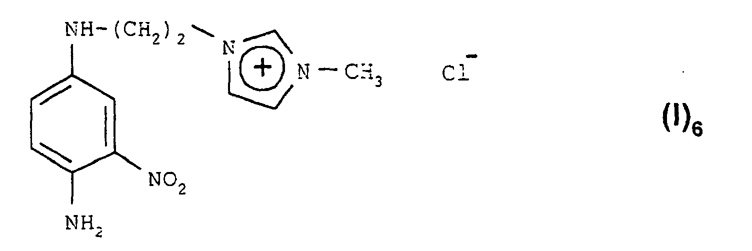 i.e. chlorure du 1-[2-(4-amino-3-nitro-phénylamino)-éthyl]-3-méthyl-3H-imidazol-1-ium, 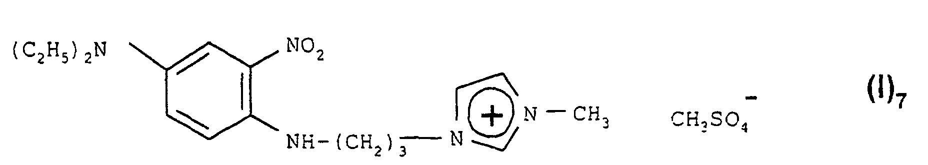 i.e. méthylsulfate du 3-[3-(4-diéthylamino-2-nitro-phénylamino]-propyl]-1-méthyl-3H-imidazol-1-ium, 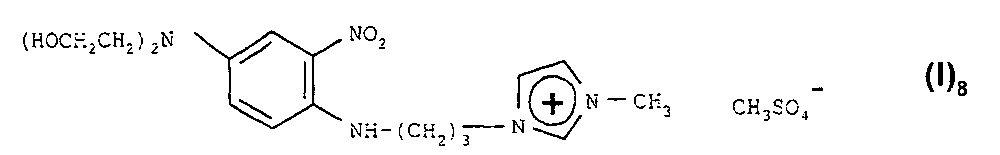 i.e. méthylsulfate du 3-[3-{4-[bis-(2-hydroxyéthyl)-amino]-2-nitro-phénylamino}-propyl]-1-méthyl-3H-imidazol-1-ium, 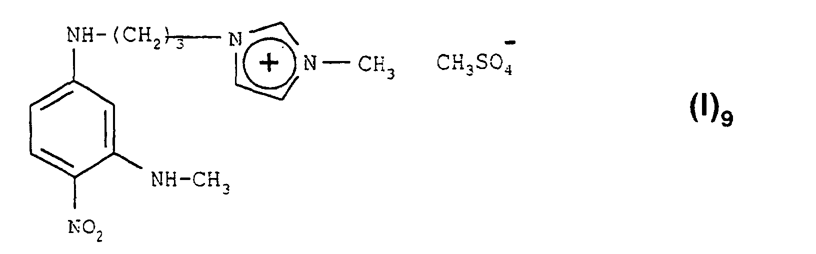 i.e. méthylsulfate du 1-méthyl-3-[3-(3-méthylamino-4-nitro-phénylamino)-propyl]-3-H-imidazol-1-ium, 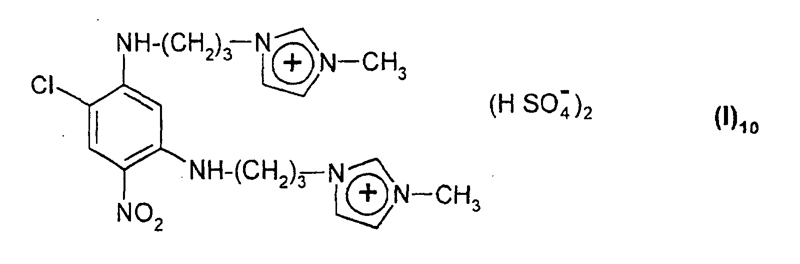 i.e. disulfate acide de 3-(3-{2-chloro-5-[3-(3-méthyl-3H-imidazol-1-ium)-propylamino]-4-nitro-phénylamino}-propyl)-1-méthyl-3H-imidazol-1-ium, 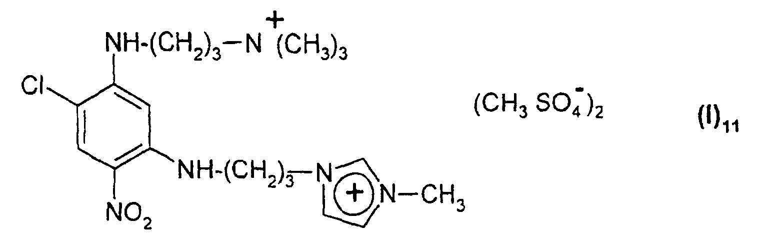 i.e. diméthylsulfate de 3-(3-{4-chloro-5-[3-(triméthyl-ammonium)-propylamino]-2-nitro-phénylamino}-propyl)-1-méthyl-3H-imidazol-1-ium, 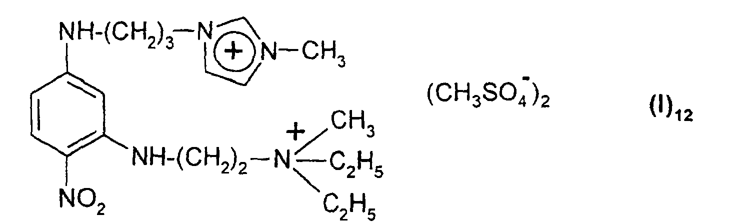 i.e. diméthylsulfate de 3-(3-{3-[2-(diéthyl-méthyl-ammonium)-éthylamino]-4-nitro-phenylamino}-propyl)-1-méthyl-3H-imidazol-1-ium, 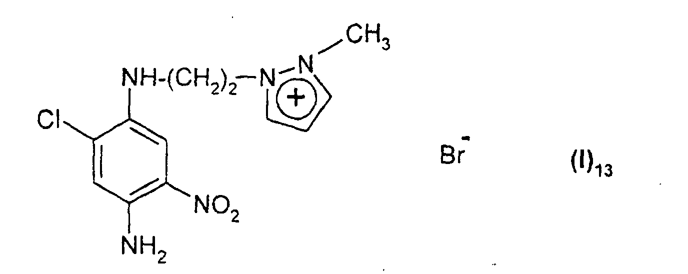 i.e. bromure du 1-[2-(4-amino-2-chloro-5-nitro-phénylamino)-éthyl]-2-méthyl-2H-pyrazol-1-ium, 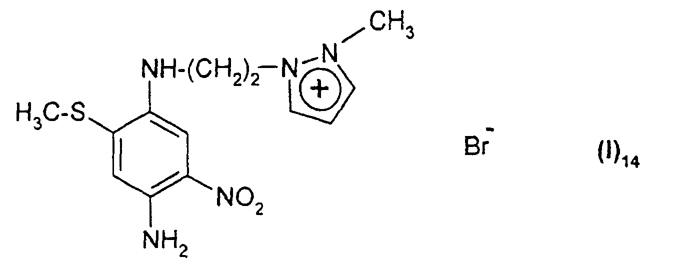 i.e. bromure du 1-[2-(4-amino-2-méthylsulfanyl-5-nitro-phénylamino)-éthyl]-2-méthyl-2H-pyrazol-1-ium, 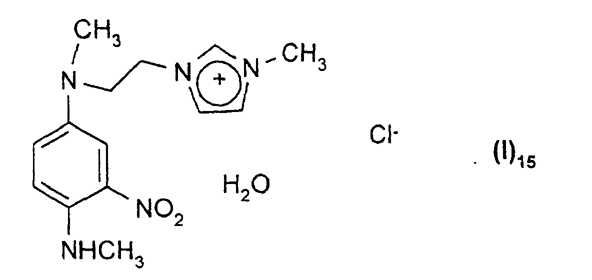 i.e. chlorure de 3-méthyl-1-{2-[méthyl-(4-méthylamino-3-nitro-phényl)-amino]-éthyl}-3H-imidazol-1-ium, monohydrate.

Les composés de formule (I) conformes à l'invention peuvent être facilement obtenus selon des méthodes généralement bien connues de l'état de la technique pour l'obtention des amines quaternisées, par exemple :
- en un temps, par condensation d'une nitrophénylènediamine comportant un radical halogénoalkyle avec un composé porteur d'un radical amine tertiaire, ou par condensation d'une nitrophénylènediamine comportant un radical amine tertiaire avec un composé porteur d'un radical halogénoalkyle ;
- ou en deux temps, par condensation d'une nitrophénylènediamine comportant un radical halogénoalkyle avec un composé porteur d'une amine secondaire, ou par condensation d'une nitrophénylènediamine halogénée avec une amino(di-substituée)alkyleamine, suivie d'une quaternisation avec un agent alkylant.

L'étape de quaternisation est, généralement par commodité, la dernière étape de la synthèse, mais peut intervenir plus tôt dans la suite des réactions conduisant à la préparation des composés de formule (I).

L'invention a aussi pour objet une composition de teinture pour matières kératiniques, comprenant, dans un milieu approprié pour la teinture, une quantité efficace d'au moins une nitrophénylènediamine cationique monobenzénique de formule (I) décrite ci-avant.

L'invention a également pour objet une composition pour la teinture directe des fibres kératiniques humaines, telles que les cheveux, comprenant dans un milieu approprié pour la teinture, une quantité efficace d'au moins une nitrophénylènediamine cationique monobenzénique telle que définie ci-avant par la formule (I).

L'invention a pour autre objet l'utilisation des nitrophénylènediamines cationiques monobenzéniques de formule (I), à titre de colorants directs, dans des, ou pour la préparation de, compositions tinctoriales pour matières kératiniques, en particulier pour fibres kératiniques humaines telles que les cheveux.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

La ou les nitrophénylènediamine(s) cationique(s) monobenzénique(s) de formule (I) conformes à l'invention et/ou le ou leurs sels d'addition avec un acide représentent de préférence de 0,005 à 12% en poids environ du poids total de la composition tinctoriale, et encore plus préférentiellement de 0,05 à 6% en poids environ de ce poids.

Les nitrophénylènediamines cationiques monobenzéniques de formule (I) conformes à l'invention peuvent également servir, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les nitrophénylènediamines cationiques monobenzéniques de formule (I), d'autre(s) colorant(s) direct(s) classiquement utilisés, et notamment, des colorants nitrés benzéniques autres que les nitrophénylènediamines cationiques de formule (I) selon la présente invention, les nitrodiphénylamines, les éthers de phénol nitrés ou les nitrophénols, des nitropyridines, des colorants anthraquinoniques, des nitroanilines, des colorants mono- ou di-azoïques, triarylméthaniques, aziniques, acridiniques et xanthéniques, ou encore des colorants métallifères.
La proportion de tous ces autres colorants directs d'addition peut varier entre environ 0,05 et 10% en poids par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Les solvants peuvent être présents dans des proportions de préférence comprises entre 1 et 40% en poids par rapport au poids total de la composition tinctoriale, et plus préférentiellement encore entre 5 et 30% en poids environ. On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actits bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition. On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier entre 3 et 12 environ et de préférence entre 5 et 11 environ. Il peut être ajusté à la valeur désirée au moyen d'agents acidifiants ou alcalinisants ou de tampons habituellement utilisés en teinture des matières kératiniques.

Les agents acidifiants sont classiquement des acides minéraux ou organiques comme par exemple les acides chlorhydrique, orthophosphorique, sulfurique, les acides carboxyliques comme les acide acétique, tartrique, citrique, lactique et sulfonique.
Parmi les tampons, on peut citer par exemple, le phosphate diacide de potassium/hydroxyde de sodium.
Parmi les agents alcalinisants, on peut citer à titre d'exemple, l'ammoniaque, les carbonates alcalins, les alcanolamines telles que les mono- di- et tri-éthanolamines et leurs dérivés, les hydroxydes de sodium ou de potassium, et les composés de formule : 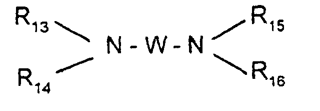 dans laquelle, W est un reste propylène éventuellement substitué par un groupement hydroxyle ou un radical alkyle en C₁-C₄ ; R₁₃ R₁₄, R₁₅ et R₁₆, simultanément ou indépendamment l'un de l'autre, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquide, de crème, de gel ou sous toute autre forme appropriée pour réaliser une teinture des matières kératiniques, et plus particulièrement des fibres kératiniques humaines et notamment des cheveux. En particulier, elle peut être conditionnée sous pression en flacon aérosol en présence d'un agent propulseur et former une mousse.

Un autre objet de la présente invention porte sur un procédé de teinture des fibres kératiniques, en particulier des fibres kératiniques humaines telles que les cheveux, par coloration directe, consistant à laisser agir une composition tinctoriale renfermant au moins une nitrophénylènediamine cationique monobenzénique de formule (I) sur les fibres kératiniques sèches ou humides. On peut utiliser la composition selon l'invention en tant que composition non rincée, c'est-à-dire qu'après application de la composition sur les fibres, on sèche sans rinçage intermédiaire.
Dans les autres modes d'application, on laisse agir la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets illustrant l'invention vont maintenant être donnés.

**EXEMPLES DE PREPARATION**

EXEMPLE 1 : **Préparation du composé de formule (I)**_{**1**} **bromure de 1-[3-(4-amino-2-chloro-5-nitro-phénylamino)-propyl]-3-méthyl-3H-imidazol-1-ium,**

**(charge délocalisée dans le cycle imidazolique).**

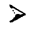 **1**^{**ère**} **étape** :

### synthèse du N-(4-amino-2-chloro-5-nitro-phényl)-N-(3-bromo-propyl)- benzènesulfonamide.

Dans un réacteur, on a chauffé au bain marie bouillant la suspension de 98,3g (0,3 mole) de N-(4-amino-2-chloro-5-nitro-phényl)-benzènesulfonamide (RN 84741-80-0) et de 25,3g d'oxyde de calcium dans 250 ml de diméthylformamide. Sous agitation, on a ajouté d'un seul coup 61,2 ml (0,6 mole) de 1,3-dibromo-propane et prolongé le chauffage pendant une heure.
Le milieu réactionnel a été filtré chaud et coulé dans 3kg d'eau glacée ; la gomme qui a précipité a été décantée et extraite à l'acétate d'éthyle.
La phase acétate d'éthyle a été séchée sur sulfate de sodium, filtrée et évaporée à sec sous pression réduite.
Le composé obtenu a été purifié par passage sur colonne de gel de silice (gradient d'heptane et d'acétate d'éthyle).
On a obtenu 55,8g de cristaux jaunes qui fondaient à 116°C (Kofler) et dont l'analyse élémentaire calculée pour C₁₅H₁₅N₃O₄SBrCl était :

| **%** | **C** | **H** | **N** | **O** | **S** | **Br** | **Cl** |
|---|---|---|---|---|---|---|---|
| Calculé | 40,15 | 3,37 | 9,36 | 14,26 | 7,15 | 17,81 | 7,90 |
| Trouvé | 40,24 | 3,37 | 9,32 | 14,53 | 6,50 | 17,58 | 7,69 |

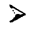 **2**^{**ème**} **étape** :

### synthèse du N1-(3-bromo-propyl)-2-chloro-5-nitro-benzène-1,4-diamine.

Le composé obtenu à l'étape 1 ci-dessus (55,8g - 0,125 mole) a été introduit par portions dans 170 ml d'acide sulfurique à 98% vivement agité et a été maintenu entre 15°C et 20°C par un bain de glace.
A la fin de l'addition, la solution a été agitée une heure supplémentaire à 15-20°C. On a ensuite versé la solution sur 1kg de glace et neutralisé en partie jusqu'à pH 5 avec de l'ammoniaque à 20% ; le précipité cristallisé a été essoré, réempâté dans l'eau et séché sous vide à 40°C sur anhydride phosphorique.
On a obtenu 35,4g de cristaux rouges qui, après purification par recristallisation de l'acétate d'isopropyle bouillant, fondaient à 128°C (Kofler) et dont l'analyse élémentaire calculée pour C₉H₁₁N₃O₂BrCl était :

| % | **C** | **H** | **N** | **O** | **Br** | **Cl** |
|---|---|---|---|---|---|---|
| Calculé | 35,03 | 3,59 | 13,62 | 10,37 | 25,90 | 11,49 |
| Trouvé | 35,13 | 3,65 | 13,62 | 10,27 | 25,69 | 11,49 |

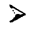 **3**^{**ème**} **étape** :

### quaternisation du composé préparé à la 2^{ème} étape.

On a réalisé la suspension de 12,3g (0,04 mole) de N1-(3-bromo-propyl)-2-chloro-5-nitro-benzène-1,4-diamine obtenu à l'étape 2 et de 3,9g (0,048mole) de 1-méthyl-1H-imidazole (RN 616-47-7) dans 40 ml de toluène.
On a chauffé sous agitation au reflux du toluène pendant 4 heures, essoré bouillant et réempâté deux fois dans l'acétate d'éthyle puis dans l'éthanol absolu. Après séchage à 40°C sous vide, on a obtenu 15,0g de cristaux rouge-foncé de bromure de 1-[3-(4-amino-2-chloro-5-nitro-phénylamino)-propyl]-3-methyl-3H-imidazol-1-ium fondant à plus de 260°C (Kofler) et dont l'analyse élémentaire calculée pour C₁₃H₁₇N₅O₂BrCl était :

| % | **C** | **H** | **N** | **O** | **S** | **Br** |
|---|---|---|---|---|---|---|
| Calculé | 39,87 | 4,39 | 17,93 | 8,19 | 20,45 | 9,07 |
| Trouvé | 39,86 | 4,39 | 18,26 | 8,14 | 20,39 | 8,94 |

**EXEMPLE 2 :** **Préparation du composé de formule (I)**_{**2**} **chlorure de 1-[2-(4-amino-2-chloro-5-nitro-phénylamino)-éthyl]-3-méthyl-3H-imidazol-1-ium.**

**(charge délocalisée dans le cycle imidazolique).**

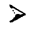 **1**^{**ère**} **étape** :

### synthèse du N-(4-amino-2-chloro-5-nitro-phényl)-N-(2-chloro-éthyl)-benzènesulfonamide.

On a suivi le mode opératoire décrit à l'exemple 1 (1^{ère} étape).
A partir de 98,3g (0,3 mole) de N-(4-amino-2-chloro-5-nitro-phényl)-benzènesulfonamide (RN 84741-80-0) et de 59,3g (0,6 mole) de 1,2-dichloroéthane on a obtenu 80,2g de cristaux jaunes qui, après purification par recristallisation de l'acétate d'éthyle bouillant, fondaient à 144°C (Kofler) et dont l'analyse élémentaire calculée pour C₁₄H₁₃N₃O₄Cl₂ était :

| % | **C** | **H** | **N** | **O** | **S** | **Cl** |
|---|---|---|---|---|---|---|
| Calculé | 43,09 | 3,36 | 10,77 | 16,40 | 8,22 | 18,17 |
| Trouvé | 43,28 | 3,55 | 10,76 | 16,39 | 7,54 | 17,95 |

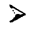 **2**^{**ème**} **étape** :

### synthèse du 2-chloro-N1-(2-chloro-éthyl)-5-nitro-benzène-1,4-diamine

On a suivi le mode opératoire décrit à l'exemple 1 (2^{ème} étape).
A partir de 79,5g (0,203 mole) de N-(4-amino-2-chloro-5-nitro-phényl)-N-(2-chloro-éthyl)-benzènesulfonamide obtenu à l'étape précédente, on a obtenu 45,0g de cristaux brun-rouge qui, après purification par recristallisation du toluène au reflux, fondaient à 117°C (Kofler) et dont l'analyse élémentaire calculée pour C₈H₉N₃O₂Cl₂ était :

| % | **C** | **H** | **N** | **O** | **Cl** |
|---|---|---|---|---|---|
| Calculé | 38,42 | 3,63 | 16,80 | 12,80 | 28,35 |
| Trouvé | 38,85 | 3,68 | 16,70 | 13,47 | 27,40 |

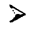 **3**^{**ème**} **étape** :

### quaternisation du composé obtenu à la 2^{ème} étape

On a suivi le mode opératoire décrit à l'exemple 1 (3^{ème} étape).
A partir de 10,0g (0,04 mole) de 2-chloro-N1-(2-chloro-éthyl)-5-nitro-benzène-1,4-diamine obtenu à la 2^{ème} étape et de 3,9g (0,048 mole) de 1-méthyl-1H-imidazole (RN 616-47-7) on a obtenu 6,2g de cristaux rouge-brique qui fondaient à 221°C (Kofler) et dont l'analyse élémentaire calculée pour C₁₂H₁₅N₅O₂Cl₂ + ½ H₂O était :

| % | **C** | **H** | **N** | **O** | **Cl** |
|---|---|---|---|---|---|
| Calculé | 42,24 | 4,73 | 20,53 | 11,72 | 20,78 |
| Trouvé | 42,83 | 4,56 | 20,49 | 11,50 | 20,87 |

**EXEMPLE 3** : **Préparation du composé de formule (I)**_{**3**} **bromure de 1-[2-(4-amino-2-chloro-5-nitro-phénylamino)-éthyl]-3-méthyl-3H-imidazol-1-ium.**

**(charge délocalisée dans le cycle imidazolique).**

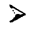 **1**^{**ère**} **étape** :

### synthèse du N-(4-amino-2-chloro-5-nitro-phényl)-N-(2-bromo-éthyl)-benzènesulfonamide.

On a suivi le mode opératoire décrit à l'exemple 1 (1^{ère} étape).
A partir de 145,5g (0,44 mole) de N-(4-amino-2-chloro-5-nitro-phényl)-benzènesulfonamide (RN 84741-80-0) et de 115 ml (1,32 moles) de 1,2-dibromo-éthane on a obtenu des cristaux jaunes qui, après purification par recristallisation de l'alcool éthylique 96° au reflux, fondaient à 115°C (Kofler) et dont l'analyse élémentaire calculée pour C₁₄H₁₃N₃O₄BrCl était :

| % | **C** | **H** | **N** | **O** | **S** |
|---|---|---|---|---|---|
| Calculé | 38,68 | 3,01 | 9,67 | 14,72 | 7,38 |
| Trouvé | 38,83 | 3,15 | 9,55 | 14,87 | 6,55 |

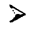 **2**^{**ème**} **étape** :

### synthèse du N1-(2-bromo-éthyl)-2-chloro-5-nitro-benzène-1,4-diamine.

On a suivi le mode opératoire décrit à l'exemple 1 (2^{ème} étape).
A partir de 191,0g (0,44 mole) de N-(4-amino-2-chloro-5-nitro-phényl)-N-(2-bromo-éthyl)- benzènesulfonamide obtenu à l'étape précédente, on a obtenu 114,5g de cristaux rouge-foncé qui, après purification par recristallisation de l'acétate d'isopropyle au reflux, fondaient à 120°C (Kofler) et dont l'analyse élémentaire calculée pour C₈H₉N₃O₂BrCl était :

| % | **C** | **H** | **N** | **O** | **Br** | **Cl** |
|---|---|---|---|---|---|---|
| Calculé | 32,62 | 3,08 | 14,27 | 10,86 | 27,13 | 12,04 |
| Trouvé | 32,78 | 3,08 | 14,20 | 10,98 | 27,03 | 12,30 |

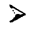 **3**^{**ème**} **étape** :

### quaternisation du composé obtenu à la 2^{ème} étape.

On a suivi le mode opératoire décrit à l'exemple 1 (3^{ème} étape).
A partir de 11,8g (0,04 mole) de N1-(2-bromo-éthyl)-2-chloro-5-nitro-benzène-1,4-diamine obtenu à l'étape précédente et de 3,9g (0,048 mole) de 1-méthyl-1H-imidazole (RN 616-47-7) on a obtenu 11,9g de cristaux rouge-brique qui fondaient à 224°C (Kofler) et dont l'analyse élémentaire calculée pour C₁₂H₁₅N₅O₂BrCl était :

| % | **C** | **H** | **N** | **O** | **Br** | **Cl** |
|---|---|---|---|---|---|---|
| Calculé | 38,27 | 4,01 | 18,59 | 8,50 | 21,21 | 9,41 |
| Trouvé | 38,11 | 4,04 | 18,33 | 8,79 | 20,87 | 9,48 |

**EXEMPLE 4** : **Préparation du composé de formule (I)**_{**4**} **bromure de 1-[2-(4-amino-2-méthyl-5-nitro-phénylamino)-éthyl]-3-méthyl-3H-imidazol-1-ium.**

**(charge délocalisée dans le cycle imidazolique).**

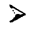 **1**^{**ère**} **étape** :

### synthèse du N-(4-amino-2-méthyl-5-nitro-phényl)-N-(2-bromo-éthyl)-4-méthyl-benzènesulfonamide.

On a suivi le mode opératoire décrit à l'exemple 1 (1^{ère} étape).
A partir de 57,0g (0,177 mole) de N-(4-amino-2-méthyl-5-nitro-phényl)-4-méthyl-benzènesulfonamide (RN 82576-78-1) et de 38,2 ml (0,44 mole) de 1,2-dibromo-éthane on a obtenu 66,0g de cristaux jaunes qui, après purification par recristallisation de l'acétate d'éthyle au reflux, fondaient à 159°C (Kofler) et dont l'analyse élémentaire calculée pour C₁₆H₁₈N₃O₄SBr était :

| % | **C** | **H** | **N** | **O** | **S** | **Br** |
|---|---|---|---|---|---|---|
| Calculé | 44,87 | 4,24 | 9,81 | 14,94 | 7,49 | 18,66 |
| Trouvé | 44,79 | 4,25 | 9,62 | 14,93 | 7,05 | 18,48 |

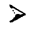 **2**^{**ème**} **étape** :

### synthèse du N1-(2-bromo-éthyl)-2-méthyl-5-nitro-benzène-1,4-diamine.

On a suivi le mode opératoire décrit à l'exemple 1 (2^{ème} étape).
A partir de 66,0g (0,154 mole) de N-(4-amino-2-méthyl-5-nitro-phényl)-N-(2-bromo-éthyl)-4-méthyl-benzènesulfonamide obtenu à l'étape précédente, on a obtenu 36,0g de cristaux brun-rouge qui, après purification par recristallisation de l'acétate d'isopropyle au reflux, fondaient à 120°C (Kofler) et dont l'analyse élémentaire calculée pour C₉H₁₂N₃O₂Br était :

| % | **C** | **H** | **N** | **O** | **Br** |
|---|---|---|---|---|---|
| Calculé | 39,44 | 4,41 | 15,33 | 11,67 | 29,15 |
| Trouvé | 38,55 | 4,44 | 5,00 | 11,60 | 29,37 |

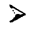 **3**^{**ème**} **étape** :

### quaternisation du composé obtenu à la 2^{ème} étape.

On a suivi le mode opératoire décrit à l'exemple 1 (3^{ème} étape).
A partir de 11,0g (0,04 mole) de N1-(2-bromo-éthyl)-2-méthyl-5-nitro-benzène-1,4-diamine obtenu à l'étape précédente et de 3,9g (0,048 mole) de 1-méthyl-1H-imidazole (RN 616-47-7) dans l'isobutanol, on a obtenu 9,9g de cristaux brun-rouge qui fondaient à 200°C (Kofler) et dont l'analyse élémentaire calculée pour C₁₃H₁₈N₅O₂Br était :

| % | **C** | **H** | **N** | **O** | **Br** |
|---|---|---|---|---|---|
| Calculé | 43,83 | 5,09 | 19,66 | 11,67 | 22,43 |
| Trouvé | 45,37 | 5,24 | 19,45 | 10,18 | 19,78 |

**EXEMPLE 5** : **Préparation du composé de formule (I)**_{**5**} **bromure de 1-[2-(4-amino-2-méthylsulfanyl-5-nitro-phénylamino)-éthyl]-3-méthyl-3H-imidazol-1-ium.**

**(charge délocalisée dans le cycle imidazolique).**

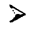 **1**^{**ère**} **étape** :

### synthèse du N-(4-amino-2-méthylsulfanyl-5-nitro-phényl)-benzènesulfonamide.

Dans un réacteur, on a dissous à température ambiante 132g (0,662 mole) de 2-méthylsulfanyl-5-nitro-benzène-1,4-diamine (RN 171968-54-0) dans 400 ml de pyridine.
Sous agitation, on a coulé goutte à goutte 120,8g (0,684 mole) de chlorure de benzènesulfonyl en maintenant la réaction exothermique entre 40°C et 45°C ; en ½ heure la solution rouge est devenu jaune-orangé ; on l'a versée sur 2,7kg de glace et on a acidifié avec 400 ml d'acide chlorhydrique à 36%.
Le précipité cristallisé jaune-ocre obtenu a été essoré, réempâté dans l'eau à neutralité et séché sous vide à 40°C sur anhydride phosphorique.
On a obtenu des cristaux jaunes qui, après recristallisation de l'acétonitrile au reflux, fondaient à 212°C (Kofler) et dont l'analyse élémentaire calculée pour C₁₃H₁₃N₃O₄S₂ était :

| % | **C** | **H** | **N** | **O** | **S** |
|---|---|---|---|---|---|
| Calculé | 46,01 | 3,86 | 12,38 | 18,86 | 18,89 |
| Trouvé | 45,94 | 3,86 | 12,35 | 18,61 | 18,24 |

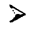 **2**^{**ème**} **étape** :

### synthèse du N-(4-amino-2-méthylsulfanyl-5-nitro-phényl)-N-(2-bromo-éthyl)-benzènesulfonamide.

On a suivi le mode opératoire décrit à l'exemple 1 (1^{ère} étape).
A partir de 116,0g (0,33 mole) de N-(4-amino-2-méthylsulfanyl-5-nitro-phényl) benzènesulfonamide obtenu à l'étape précédente et de 57,0 ml (0,66 mole) de 1,2-dibromo-éthane, on a obenu des cristaux jaunes qui, après purification par recristallisation de la méthyléthylcétone au reflux, fondaient à 216°C (Kofler) et dont l'analyse élémentaire calculée pour C₁₅H₁₆N₃O₄S₂Br était:

| % | **C** | **H** | **N** | **O** | **S** | **Br** |
|---|---|---|---|---|---|---|
| Calculé | 40,37 | 3,61 | 9,41 | 14,34 | 14,37 | 17,90 |
| Trouvé | 40,23 | 3,73 | 9,10 | 15,19 | 13,55 | 17,84 |

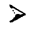 **3**^{**ème**} **étape** :

### synthèse du N1-(2-bromo-éthyl)-2-méthylsulfanyl-5-nitro-benzène-1,4-diamine.

On a suivi le mode opératoire décrit à l'exemple 1 (2^{ème} étape).
A partir de 147,3g (0,33 mole) de N-(4-amino-2-méthylsulfanyl-5-nitro-phényl)-N-(2-bromo-éthyl)-benzènesulfonamide obtenu à l'étape précédente, on a obtenu 72,0g de cristaux rouges qui, après purification par recristallisation de l'acétate d'éthyle au reflux, fondaient à 131°C (Kofler) et dont l'analyse élémentaire calculée pour C₉H₁₂N₃O₂SBr était :

| % | **C** | **H** | **N** | **O** | **S** | **Br** |
|---|---|---|---|---|---|---|
| Calculé | 35,31 | 3,95 | 13,72 | 10,45 | 10,47 | 26,10 |
| Trouvé | 35,56 | 4,06 | 13,72 | 10,57 | 10,14 | 26,39 |

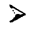 **4**^{**ème**} **étape** :

### quaternisation du composé obtenu à la 3^{ème} étape.

On a suivi le mode opératoire décrit à l'exemple 1 (3^{ème} étape).
A partir de 12,2g (0,04 mole) de N1-(2-bromo-éthyl)-2-méthylsulfanyl-5-nitro-benzène-1,4-diamine obtenu à l'étape précédente et de 3,9g (0,048 mole) de 1-méthyl-1H-imidazole (RN 616-47-7) dans le toluène, on a obtenu 11,0g de cristaux rouge-brique qui fondaient à 196°C (Kofler) et dont l'analyse élémentaire calculée pour C₁₃H₁₈N₅O₂SBr + ½ H₂O était:

| % | **C** | **H** | **N** | **O** | **S** | **Br** |
|---|---|---|---|---|---|---|
| Calculé | 39,30 | 4,82 | 17,63 | 10,07 | 8,07 | 20,11 |
| Trouvé | 40,44 | 4,69 | 17,49 | 9,44 | 8,56 | 18,96 |

### EXEMPLE 6 : Préparation du composé de formule (I)₆ chlorure de 1-[2-(4-amino-3-nitro-phényl-amino)-éthyl]-3-méthyl-3H-imidazol-1-ium.

### (charge délocalisée dans le cycle imidazolique).

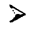 **1**^{**ère**} **étape** :
*synthèse du N-(4-amino-3-nitro-phényl)-N-(2-chloro-éthyl)-4-méthyl- benzènesulfonamide.*

On a suivi le mode opératoire décrit à l'exemple 1 (1^{ère} étape).
A partir de 23,0g (0,075 mole) de N-(4-amino-3-nitro-phényl)-4-méthyl-benzènesulfonamide (RN 59457-54-4) et de 29,7g (0,3 mole) de 1,2-dichloro-éthane on a obtenu des cristaux jaunes qui, après purification par recristallisation de l'alcool éthylique 96° au reflux, fondaient à 146°C (Kofler) et dont l'analyse élémentaire calculée pour C₁₅H₁₆N₃O₄SCl était :

| % | **C** | **H** | **N** | **O** | **S** | **Cl** |
|---|---|---|---|---|---|---|
| Calculé | 48,72 | 4,36 | 11,36 | 17,30 | 8,67 | 9,59 |
| Trouvé | 48,89 | 4,38 | 11,29 | 17,43 | 8,64 | 9,72 |

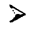 **2**^{**ème**} **étape** :

### synthèse du N4-(2-chloro-éthyl)-2-nitro-benzène-1,4-diamine et quaternisation.

On a suivi le mode opératoire décrit à l'exemple 1 (2^{ème} étape).
A partir de 136,0g (0,365 mole) de N-(4-amino-3-nitro-phényl)-N-(2-chloro-éthyl)-4-méthyl-benzènesulfonamide obtenu à l'étape précédente, on a obtenu 75,7g de cristaux brun-rouge de N4-(2-chloro-éthyl)-2-nitro-benzène-1,4-diamine qui, après purification par recristallisation du benzène au reflux, fondaient à 107°C (Kofler).

Pour la quaternisation, on a suivi le mode opératoire décrit à l'exemple 1 (3^{ème} étape).
A partir de 10,8g (0,05 mole) de N4-(2-chloro-éthyl)-2-nitro-benzène-1,4-diamine obtenu ci-avant et de 4,9g (0,06 mole) de 1-méthyl-1H-imidazole (RN 616-47-7) dans le toluène on a obtenu 11,5g de cristaux brun-rouge qui fondaient à 204°C et dont l'analyse élémentaire calculée pour C₁₂H₁₆N₅O₂Cl + ¼ H₂O était :

| % | **C** | **H** | **N** | **O** | **Cl** |
|---|---|---|---|---|---|
| Calculé | 47,69 | 5,50 | 23,17 | 11,91 | 11,73 |
| Trouvé | 47,52 | 5,50 | 23,20 | 11,98 | 11,85 |

**EXEMPLE 7 :** **Préparation du composé de formule (I)**_{**7**} **méthylsulfate de 3-[3-(4-diéthylamino-2-nitro-phénylamino)-propyl]-1-méthyl-3H-imidazol-1-ium.**

**(charge délocalisée dans le cycle imidazolique).**

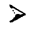 **1**^{**ère**} **étape** :

### synthèse du diéthyl-(4-fluoro-3-nitro-phényl)-amine.

Dans un mélange chauffé à 65°C, comprenant 63,0g (0,5 mole) de 4-fluoro-3-nitro-phénylamine.(RN 364-76-1) et 110g de carbonate de calcium dans 150 ml de diméthylformamide, on a coulé en 2 heures, sous agitation, 197ml (1,5 moles) de diéthylsulfate; puis on a chauffé 2 heures à 85°C-90°C.
On a filtré chaud et coulé dans 2kg d'eau glacée.
L'huile qui a décanté a été extraite à l'acétate d'éthyle ; on a séché sur sulfate de sodium, filtré et évaporé à sec sous pression réduite.
On a obtenu 46,3g d'une huile rouge-orangé qui, après purification par passage sur une colonne de gel de silice (gradient d'heptane et d'acétate d'éthyle), a cristallisé (F < 50°C - Kofler) et dont l'analyse élémentaire calculée pour C₁₀H₁₃N₂O₂F était :

| % | **C** | **H** | **N** | **F** |
|---|---|---|---|---|
| Calculé | 56,60 | 6,17 | 13,20 | 8,95 |
| Trouvé | 56,47 | 6,22 | 13,20 | 8,91 |

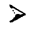 **2**^{**ème**} **étape** :

### synthèse du N4,N4-diéthyl-N1-(3-imidazol-1-yl-propyl)-2-nitro-benzène-1,4-diamine.

Sous agitation on a chauffé pendant 2 heures au reflux, un mélange de 8,8g (0,041 mole) de diéthyl-(4-fluoro-3-nitro-phényl)-amine obtenu à l'étape précédente, de 17,0g (0,136 mole) de 3-imidazol-1-yl-propylamine (RN 5036-48-6) et de 6,7ml de triéthylamine.
On a versé sur 100g d'eau glacée ; l'huile qui a décanté a été extraite à l'acétate d'éthyle. Puis on a séché sur sulfate de sodium, filtré et évaporé à sec sous pression réduite.
On a obtenu 12,8g d'une huile violette qui, après purification par passage sur une colonne de gel de silice (gradient d'heptane et d'acétate d'éthyle), a cristallisé (F > 260°C - Kofler) et dont l'analyse élémentaire calculée pour C₁₆H₂₃N₅O₂ était :

| % | **C** | **H** | **N** | **O** |
|---|---|---|---|---|
| Calculé | 60,55 | 7,30 | 22,07 | 10,08 |
| Trouvé | 60,39 | 7,29 | 22,05 | 10,20 |

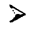 **3**^{**ème**} **étape** :

### quaternisation du composé obtenu à la 2^{éme} étape.

On a réalisé la suspension de 6,3g (0,02 mole) de N4,N4-diéthyl-N1-(3-imidazol-1-yl-propyl)-2-nitro-benzène-1,4-diamine obtenu à l'étape précédente et 2,09 ml (0,022mole) de diméthylsulfate dans 100ml d'acétate d'éthyle et on a laissé 3 heures à température ambiante, sous agitation.
Le précipité cristallisé obtenu a été essoré, lavé plusieurs fois dans l'acétate d'éthyle et séché à 50°C sous vide.
On a obtenu 8,2g de cristaux violet-foncé qui fondaient à 101°C (Kofler) et dont l'analyse élémentaire calculée pour C₁₈H₂₉N₅O₆S était :

| % | **C** | **H** | **N** | **O** | **S** |
|---|---|---|---|---|---|
| Calculé | 48,75 | 6,59 | 15,79 | 21,64 | 7,23 |
| Trouvé | 48,50 | 6,66 | 15,79 | 22,16 | 7,21 |

**EXEMPLE 8** : **Préparation du composé de formule (I)**₈ **méthylsulfate de 5-(3-{4-[bis-(2-hydroxy-éthyl)-amino]-2-nitro-phénylamino}-propyl)-1-méthyl-3H-imidazol-1-ium.**

**(charge délocalisée dans le cycle imidazolique).**

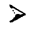 **1**^{**ère**} **étape** :

### synthèse du 2-{(2-hydroxyéthyl)-[4-(3-imidazol-1-yl-propyl-amino)-3-nitro-phényl]-amino}-éthanol.

On a utilisé le mode opératoire décrit à l'exemple 7 (2^{ème} étape).
A partir de 29,3g (0,12 mole) de 2-[(4-fluoro-3-nitro-phényl)-(2-hydroxyéthyl) amino]-éthanol (RN 29705-38-2) et de 50,0g (0,4 mole) de 3-imidazol-1-yl-propylamine (RN 5036-48-6) on a obtenu 39,0g de cristaux violets qui, après purification par recristallisation de l'alcool éthylique 96° au reflux, fondaient à 141°C (Kofler) et dont l'analyse élémentaire calculée pour C₁₆H₂₃N₅O₄ était :

| % | **C** | **H** | **N** | **O** |
|---|---|---|---|---|
| Calculé | 55,00 | 6,64 | 20,04 | 18,32 |
| Trouvé | 54,77 | 6,37 | 20,35 | 18,39 |

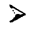 **2**^{**ème**} **étape** :

### quaternisation du composé obtenu à la 1^{ème} étape.

On a suivi le mode opératoire décrit à l'exemple 7 (3^{ème} étape).
A partir de 14,0g (0,04 mole) de 2-{(2-hydroxyéthyl)-[4-(3-imidazol-1-yl-propylamino)-3-nitro-phényl]-amino}-éthanol obtenu à l'étape précédente et de 4,2 ml (0,044 mole) de diméthylsulfate, on a obtenu 17,9g d'une huile bleu-violet dont l'analyse élémentaire calculée pour C₁₈H₂₉N₅O₈S + ½ H₂O était :

| % | **C** | **H** | **N** | **O** | **S** |
|---|---|---|---|---|---|
| Calculé | 44,62 | 6,24 | 14,45 | 28,07 | 6,62 |
| Trouvé | 44,49 | 6,54 | 13,88 | 28,22 | 6,67 |

**EXEMPLE 9** : **Préparation du composé de formule (I)**_{**9**} **methylsulfate de 1-méthyl-3-[3-(3-méthylamino-4-nitro-phénylamino)-propyl]-3H-imidazol-1-ium.**

**(charge délocalisée dans le cycle imidazolique).**

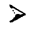 **1**^{**ère**} **étape** :

### synthèse du N1-(3-imidazol-1-yl-propyl)-N3-méthyl-4-nitro-benzène-1,3-diamine.

On a suivi le mode opératoire décrit à l'exemple 7 (2^{ème} étape).
A partir de 37,3g (0,2 mole) de (5-chloro-2-nitro-phényl)-méthyl-amine (RN 35966-84-8) et de 37,5g (0,3 mole) de 3-imidazol-1-yl-propylamine (RN 5036-48-6) on a obtenu des cristaux jaune (52,0g) qui, après purification par recristallisation de l'alcool éthylique 96° au reflux, fondaient à 145°C (Kofler) et dont l'analyse élémentaire calculée pour C₁₃H₁₇N₅O₂ était :

| % | **C** | **H** | **N** | **O** |
|---|---|---|---|---|
| Calculé | 56,72 | 6,22 | 25,44 | 11,62 |
| Trouvé | 56,64 | 6,34 | 25,37 | 11,66 |

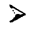 **2**^{**ème**} **étape** :

### quaternisation du composé obtenu à la 1^{ème} étape.

On a suivi le mode opératoire décrit à l'exemple 7 (3^{ème} étape).
A partir de 8,2g (0,03 mole) de N1-(3-imidazol-1-yl-propyl)-N3-méthyl-4-nitro-benzène-1,3-diamine obtenu à l'étape précédente et de 3,2 ml (0,034 mole) de diméthylsulfate on a obtenu 11,9g de cristaux jaunes qui fondaient à 120°C (Kofler) et dont l'analyse élémentaire calculée pour C₁₅H₂₃N₅O₅S était :

| % | **C** | **H** | **N** | **O** | **S** |
|---|---|---|---|---|---|
| Calculé | 44,88 | 5,77 | 17,45 | 23,91 | 7,99 |
| Trouvé | 44,75 | 5,80 | 17,61 | 24,12 | 7,90 |

**EXEMPLE 10** : **Préparation du composé de formule (I)**_{**10**} disulfate acide de 3-(3-{2-Chloro-5-[3-(3-méthyl-3H-imidazol-1-ium)-propylamino]-4-nitro-phénylamino}-propyl)-1-méthyl-3H-imidazol-1-ium.

**(charge délocalisée dans le cycle imidazolique).**

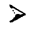 **1**^{**ère**} **étape** :

### synthèse du 4-chloro-N1, N3-bis-(3-imidazol-1-yl-propyl)-6-nitro-benzène-1,3-diamine.

Sous agitation, on a chauffé pendant 9 heures au reflux, un mélange de 113,2g (0,5 mole) de 1,2,4-trichloro-5-nitrobenzène (RN 89-69-0), de 250,4g (2 moles) de 3-imidazol-1-yl-propylamine (RN 5036-48-6) et de 138g (1 mole) de carbonate de calcium dans 660 ml de dioxane.
On a versé dans 3,3 l d'eau glacée, essoré le précipité cristallisé, réempâté dans l'eau et séché sous vide à 50°C sur anhydride phosphorique.
Après purification par recristallisation de l'alcool absolu au reflux, on a obtenu 139,3g de cristaux jaunes qui fondaient à 108-110°C (Kofler) et dont l'analyse élémentaire calculée pour C₁₈H₂₂N₇O₂Cl + 1,5 H₂O était :

| % | **C** | **H** | **N** | **O** | **Cl** |
|---|---|---|---|---|---|
| Calculé | 50,17 | 5,85 | 22,75 | 13,00 | 8,23 |
| Trouvé | 50,57 | 6,07 | 22,85 | 13,10 | 8,00 |

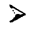 **2**^{**ème**} **étape** :

### quaternisation du composé obtenu à la 1^{ème} étape.

On a suivi le mode opératoire décrit à l'exemple 7 (3^{ème} étape).
A partir de 21,5g (0,05 mole) de 4-chloro-N1, N3-bis-(3-imidazol-1-yl-propyl)-6-nitro-benzène-1,3-diamine cristallisé avec 1,5 molécule d'eau obtenu à l'étape précédente et de 14,3g (0,113 mole) de diméthylsulfate, et après réempâtage dans l'alcool absolu chauffé à 30°C, on a obtenu 12,0g de cristaux jaunes fondant à 188-190°C (Kofler) et dont l'analyse élémentaire calculée pour C₂₀H₃₀N₇O₁₀S₂Cl était :

| % | **C** | **H** | **N** | **O** | **Cl** | **S** |
|---|---|---|---|---|---|---|
| Calculé | 38,25 | 4,81 | 15,61 | 25,47 | 5,64 | 10,21 |
| Trouvé | 37,90 | 4,88 | 15,46 | 25,42 | 5,45 | 10,23 |

### EXEMPLE 11 : Préparation du composé de formule (I)₁₅ chlorure de 3-méthyl-1-{2-[méthyl-(4-méthylamino-3-nitro-phényl)-amino]-éthyl}-3H-imidazol-1-ium, monohydrate

### (charge délocalisée dans le cycle imidazolique).

On a suivi le mode opératoire décrit à l'exemple 1 (3^{ème} étape).
A partir de 41,4g (0,17 mole) de N4-(2-chloro-éthyl)-N1,N4-diméthyl-2-nitro-benzène-1,4-diamine (RN14607-54-6) et de 41,8g (0,51 mole) de 1-méthyl-1H-imidazole (RN 616-47-7) dans 100ml de toluène, on a obtenu, après recristallisation de l'éthanol au reflux, 37,8g de cristaux violets de chlorure de 3-méthyl-1-{2-[méthyl-(4-méthylamino-3-nitro-phényl)-amino]-éthyl}-3H-imidazol-1-ium, monohydrate, qui fondaient à 135°C (Kofler) et dont l'analyse élémentaire calculée pour C₁₄H₂₀N₅O₂Cl + H₂O était :

| % | **C** | **H** | **N** | **O** | **Cl** |
|---|---|---|---|---|---|
| Calculé | 48,91 | 6,45 | 20,37 | 13,96 | 10,31 |
| Trouvé | 48,65 | 6,50 | 20,29 | 14,00 | 10,28 |

### EXEMPLES DE COMPOSITIONS TINCTORIALES

### EXEMPLE 1:

On a préparé la composition de teinture suivante :
*(toutes teneurs exprimées en grammes - M.A. désigne Matière Active)*

| | |
|---|---|
| Colorant de formule (I)₉ | 0,344 |
| Hydroxyéthylcellulose vendue sous la dénomination NATROSOL 250 MR par la société Aqualon | 0,72 |
| Alcool benzylique | 4 |
| Polyéthylèneglycol à 6 oxyde d'éthylène | 6 |
| Alkyl(C8-C10) polyglucoside en solution aqueuse à 60% M.A. vendu sous la dénomination ORAMIX CG 110 par la | |
| société Seppic | 4,5 M.A. |
| Tampon phosphate pH 9 (acide borique/chlorure de potassium/hydroxyde de sodium q.s.p | 100 |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance violet irisé.

### EXEMPLES 2 à 7:

On a préparé les six compositions de teinture suivantes :
*(toutes teneurs exprimées en grammes)*

| **EXEMPLE** | **2** | **3** | **4** | **5** | **6** | **7** |
|---|---|---|---|---|---|---|
| Composé de formule (I) ₁ | 0,298 | | | | | |
| Composé de formule (I) ₂ | | 0,356 | | | | |
| Composé de formule (I) ₃ | | | 0,377 | | | |
| Composé de formule (I) ₄ | | | | 0,332 | | |
| Composé de formule (I) ₅ | | | | | 0,391 | |
| Composé de formule (I) ₆ | | | | | | 0,385 |
| Monoéthyléther d'éthylèneglycol | 10 | 10 | 10 | 10 | 10 | 10 |
| Mélange alcool cétylstéarylique/lauryl sulfate de sodium commercialisé sous la dénomination SINNOWAX SX par la société HENKEL | 2 | 2 | 2 | 2 | 2 | 2 |
| Alcool gras linéaire (C₁₃ -C₁₅ ) oxyéthyléné (30E) vendu sous la dénomination SYNPERONIC A3 par la société I.C.I | 3 | 3 | 3 | 3 | 3 | 3 |
| Alcool gras linéaire (C₁₃ -C₁₅ ) oxyéthyléné (70E) vendu sous la dénomination SYNPERONIC A7 par la société I.C.I | 2 | 2 | 2 | 2 | 2 | 2 |
| Bromure de triméthylcétylammonium | 1,5 | 1,5 | 1,5 | 1,5 | 1,5 | 1,5 |
| Monoéthanolamine qs pH | 8 | 8 | 8 | 8 | 8 | 8 |
| Eau déminéralisée qsp | 100 | 100 | 100 | 100 | 100 | 100 |

On a appliqué chacune des compositions ci-dessus sur des mèches de cheveux gris permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 2 | légèrement violine |
| Composition de l'exemple 3 | irisé légèrement rouge |
| Composition de l'exemple 4 | cuivré rouge |
| Composition de l'exemple 5 | cuivré rouge |
| Composition de l'exemple 6 | irisé légèrement cuivré |
| Composition de l'exemple 7 | cuivré |

### EXEMPLES 8 et 9:

On a préparé les deux compositions de teinture suivantes :
*(toutes teneurs exprimées en grammes)*

| **EXEMPLE** | **8** | **9** |
|---|---|---|
| Composé de formule (I)₈ | 0,356 | |
| Composé de formule (I)₉ | | 0,344 |
| Diéthanolamide oléïque | 3 | 3 |
| Acide laurique | 1 | 1 |
| Monoéthyléther de l'éthylèneglycol | 5 | 5 |
| Hydroxyéthylcellulose | 2 | 2 |
| 2-amino-2-méthyl-1-propanol q.s pH. | 9,5 | 9,5 |
| Eau déminéralisée q.s.p | 100 | 100 |

On a appliqué chacune des compositions ci-dessus sur des mèches de cheveux gris permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 8 | violet |
| Composition de l'exemple 9 | violet |

## Revendications

1. Nitrophénylènediamines cationiques monobenzéniques de formule (I) et leurs sels d'addition avec un acide : 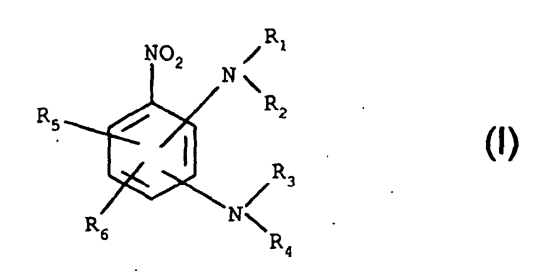 formule dans laquelle,
• **R**_{**1**}, **R**_{**2**}, **R**_{**3**}, et **R**_{**4**}, qui peuvent être identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
• **R**_{**5**}, **R**_{**6**}, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical aminoalkyle(C₁-C₆) dont l'alkyle est substitué ou non substitué par un ou plusieurs radicaux hydroxy et dont l'amine est non substituée ou substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ; un groupement OR₇ ou -SR₇ défini ci-après
• **R**_{**7**} désigne un atome d'hydrogène ; un radical alkyle en C₁-C₆; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est non substitué ou substitué par un ou plusieurs radicaux hydroxy; un radical aminoalkyle en C₁-C₆ dont l'alkyle est non substitué ou substitué par un ou plusieurs radicaux hydroxy et dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
• **Z** est choisi parmi les groupements cationiques insaturés de formule (II) suivante, et les groupements cationiques saturés de formule (III) suivante : 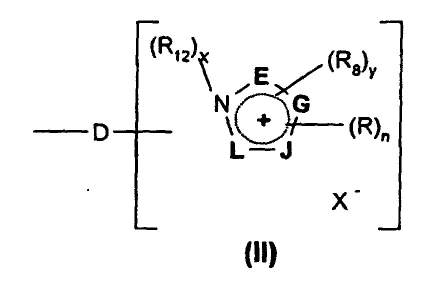 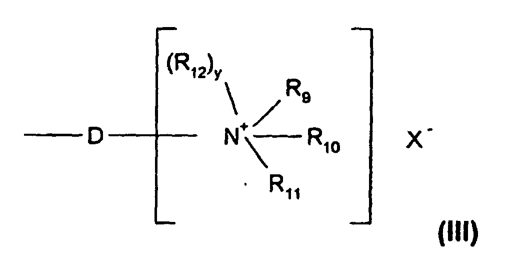
dans lesquelles :
• D est un bras de liaison qui représente une chaîne alkyle comportant de préférence de 1 à 14 atomes de carbone, linéaire ou ramifiée pouvant être substituée par un ou plusieurs radicaux hydroxyle ou alcoxy en C₁-C₆ ;
• les sommets E, G, J et L, identiques ou différents, représentent un atome de carbone ou d'azote ;
• n est un nombre entier compris entre 0 et 4 inclusivement ;
• les radicaux **R**, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₈ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ;
• R₉, R₁₀ et R₁₁, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₉, R₁₀ et R₁₁ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
• R₁₂ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes :
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₈ est porté par l'atome d'azote du cycle insaturé ; ou bien
2) lorsqu'au moins un des sommets E, G, J et L représente un atome d'azote sur lequel le radical R₈ est fixé ;
- dans les groupements cationiques de formule (III) :
- lorsque y = 0, alors le bras de liaison D est rattaché à l'atome d'azote portant les radicaux R₉ à R₁₁,
- lorsque y = 1, alors deux des radicaux R₉ à R₁₁ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
• **X**^{**-**} représente un anion monovalent ou divalent,
**étant entendu** que le nombre de groupements cationiques insaturés Z de formule (II), dans lesquels au moins un des sommets E, G, J et L représente un atome d'azote, est au moins égal à 1.

2. Nitrophénylènediamines cationiques monobenzéniques selon la revendication 1, **caractérisée par le fait que** les cycles des groupements insaturés Z de formule (II) sont choisis parmi les cycles pyrrolique, imidazolique, pyrazolique et triazolique.

3. Nitrophénylènediamines cationiques monobenzéniques selon l'une quelconque des revendications précédentes, **caractérisée par le fait que** dans la formule (III) deux des radicaux R₉, R₁₀ et R₁₁ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

4. Nitrophénylènediamines cationiques monobenzéniques selon l'une quelconque des revendications précédentes, **caractérisées par le fait que** X⁻ est choisi parmi un atome d'halogène, un hydroxyde, un hydrogènesulfate, ou un alkyl(C₁-C₆)sulfate.

5. Nitrophénylènediamines cationiques monobenzéniques selon l'une quelconque des revendications précédentes, **caractérisées par le fait qu'**elles sont choisies parmi celles de formules (I)₁ à (I)₁₅ suivantes :

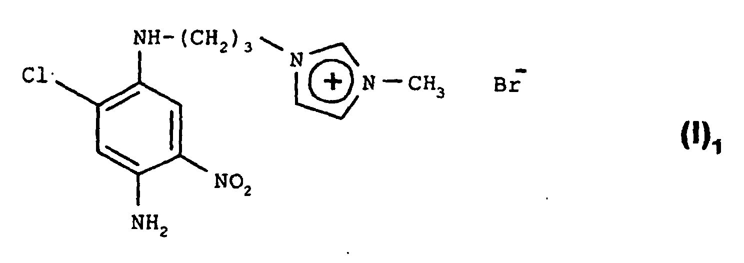

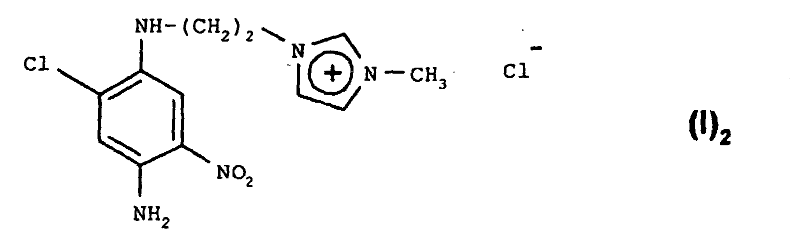

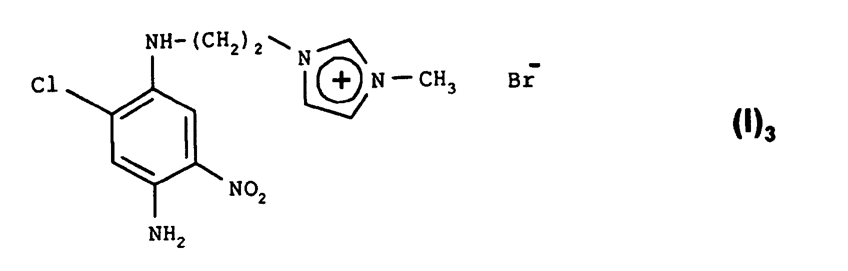

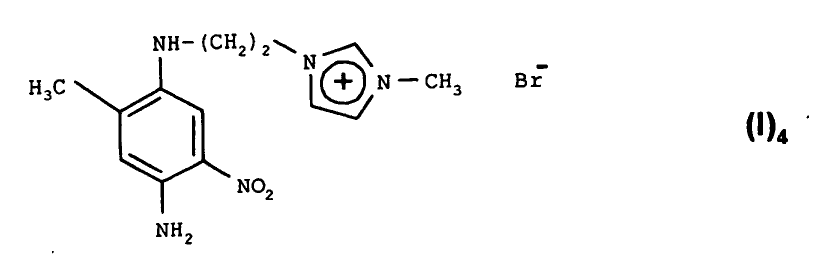

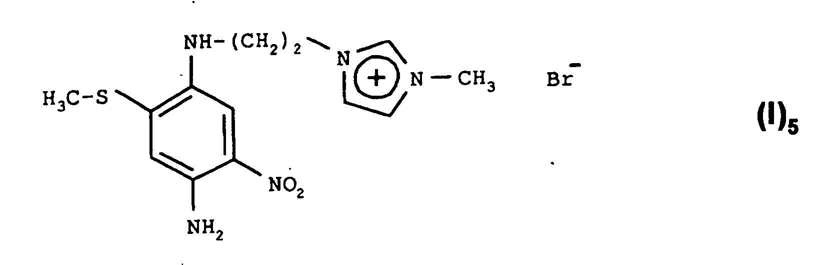

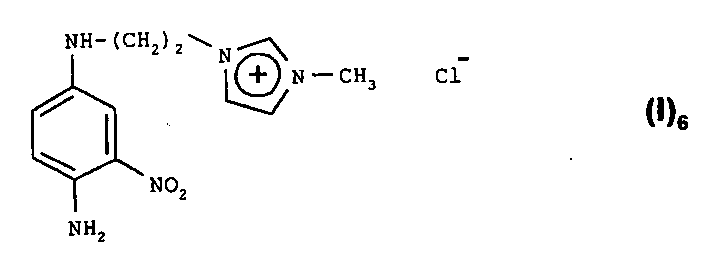

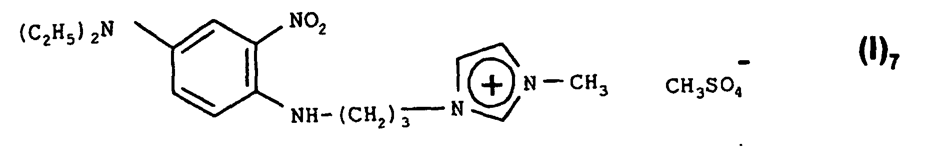

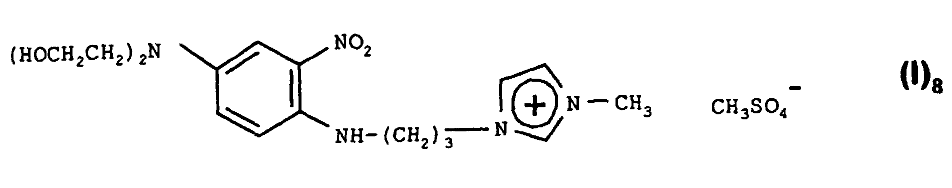

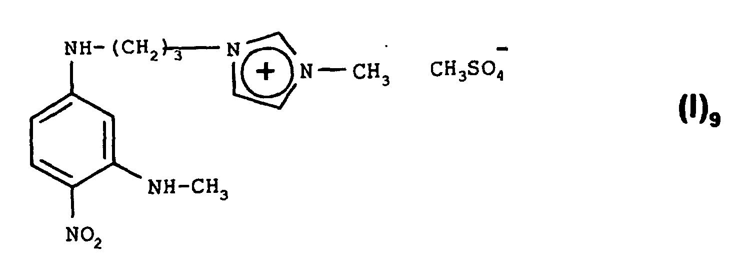

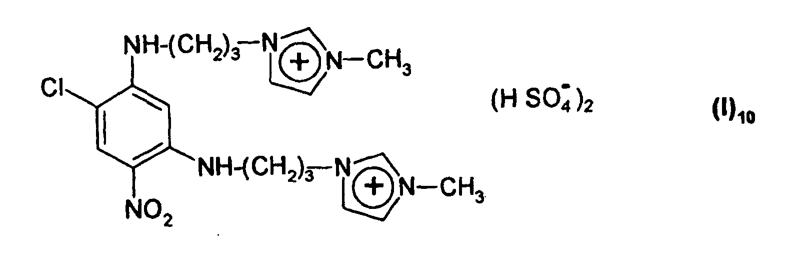

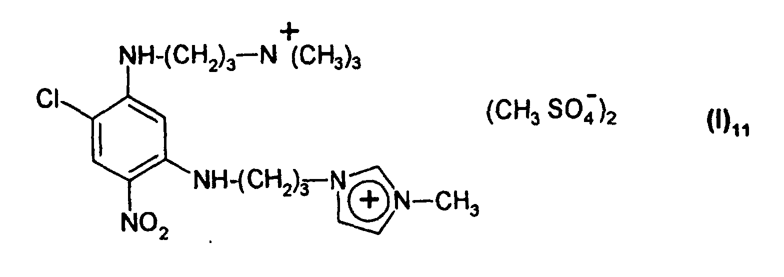

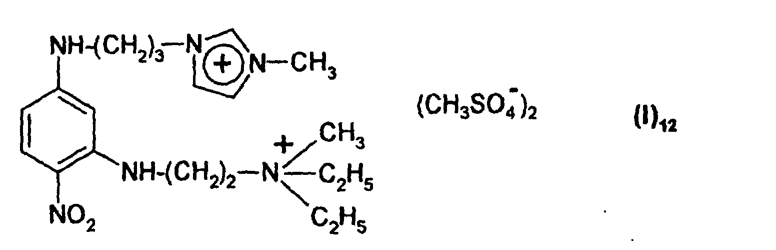

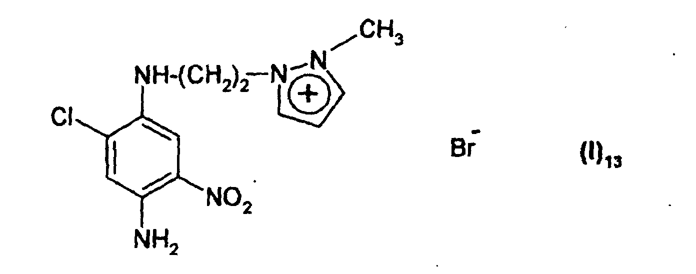

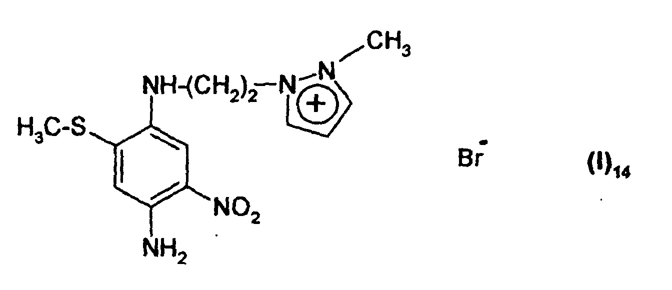

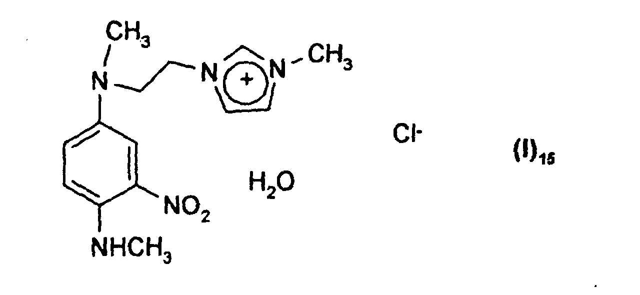

6. Nitrophénylènediamines cationiques monobenzéniques selon l'une quelconque des revendications précédentes, **caractérisées par le fait que** les sels d'addition avec un acide sont choisis parmi les chlorhydrates, les bromhydrates, les sulfates, les citrates, les succinates, les tartrates, les lactates et les acétates.

7. Utilisation des nitrophénylènediamines cationiques monobenzéniques de formule (I) telles que définies à l'une quelconque des revendications 1 à 6, à titre de colorants directs dans des, ou pour la fabrication de, compositions tinctoriales pour matières kératiniques, en particulier pour fibres kératiniques humaines et notamment les cheveux.

8. Composition de teinture pour matières kératiniques, **caractérisée par le fait qu'**elle contient, dans un milieu approprié pour la teinture, une quantité efficace d'au moins une nitrophénylènediamine cationique monobenzénique de formule (I) définie à l'une quelconque des revendications 1 à 6.

9. Composition de teinture directe pour fibres kératiniques humaines, et notamment les cheveux, **caractérisée par le fait qu'**elle contient, dans un milieu approprié pour la teinture, une quantité efficace d'au moins une nitrophénylènediamine cationique monobenzénique de formule (I) définie à l'une quelconque des revendications 1 à 6.

10. Composition selon les revendications 8 ou 9, **caractérisée par le fait qu'**elle a un pH compris entre 3 et 12.

11. Composition selon l'une quelconque des revendications 8 à 10, **caractérisée par le fait que** les nitrophénylènediamines cationiques monobenzéniques de formule (I) sont présentes dans une concentration allant de 0,005 à 12% en poids par rapport au poids total de la composition.

12. Composition selon la revendication 11, **caractérisée par le fait que** les nitrophénylènediamines cationiques monobenzéniques de formule (I) sont présentes dans une concentration allant de 0,05 à 6% en poids par rapport au poids total de la composition.

13. Composition selon l'une quelconque des revendications 8 à 12, **caractérisée par le fait qu'**elle contient d'autres colorants directs, choisis parmi des colorants nitrés benzéniques autres que les nitrophénylènediamines cationiques de formule (I) selon l'invention et tels que les nitrodiphénylamines, les nitroanilines, les éthers de phénols nitrés ou les nitrophénols, des nitropyridines, des colorants anthraquinoniques, des colorants mono- ou di-azoïques, triarylméthaniques, aziniques, acridiniques, xanthéniques, ou des colorants métallifères.

14. Composition selon l'une quelconque des revendications 8 à 13, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 1 et 40% en poids par rapport au poids total de la composition.

15. Procédé de teinture des fibres kératiniques et en particulier des fibres kératiniques humaines telles que les cheveux, par coloration directe, **caractérisé par le fait qu'**on applique la composition tinctoriale définie à l'une quelconque des revendications 8 à 14, sur les fibres kératiniques sèches ou humides, et qu'on sèche ces fibres sans rinçage intermédiaire.

16. Procédé de teinture des fibres kératiniques et en particulier des fibres kératiniques humaines telles que les cheveux, par coloration directe, **caractérisé par le fait qu'**on applique la composition tinctoriale définie à l'une quelconque des revendications 8 à 14, sur les fibres kératiniques sèches ou humides, et qu'après avoir laissé agir la composition pendant 3 à 60 minutes environ, on rince les fibres, on les lave éventuellement, on les rince à nouveau puis on les sèche.

## Patentansprüche

1. Kationische Nitro-phenylendiamine auf Monobenzolbasis der Formel (I) und deren Additionssalze mit einer Säure: 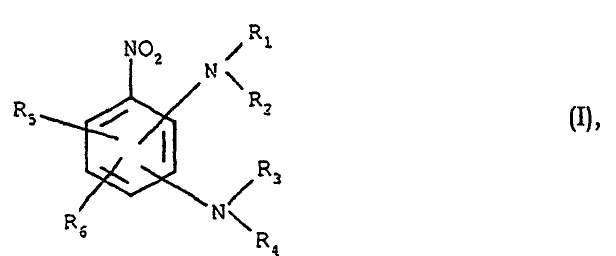 wobei in der Formel bedeuten:
• die Gruppen R₁, R₂, R₃ und R₄, die identisch oder voneinander verschieden sein können, ein Wasserstoffatom; eine nachfolgend definierte Gruppe Z; C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; N,N-Dialkyl-(C₁₋₆)carbamoyl(C₁₋₆); Thiocarbamoylalkyl(C₁₋₆); Trifluoralkyl(C₁₋₆); Sulfoalkyl(C₁₋₆); Alkyl(C₁₋₆)carboxyalkyl(C₁₋₆); Alkyl-(C₁₋₆)sulfinylalkyl(C₁₋₆); Aminosulfonylalkyl(C₁₋₆); N-Alkyl(C₁₋₆)-aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); Aminoalkyl(C₁₋₆), wobei die Alkylgruppe unsubstituiert vorliegt oder mit einer oder mehreren Hydroxygruppen substituiert ist; Aminoalkyl(C₁₋₆), wobei die Alkylgruppe unsubstituiert vorliegt oder mit einer oder mehreren Hydroxygruppen substituiert ist und wobei die Aminogruppe mit einer oder zwei Gruppen substituiert ist, die gleich oder verschieden sind und unter den Gruppen Alkyl, Monohydroxyalkyl(C₁₋₆), Polyhydroxyalkyl(C₂₋₆), Alkyl(C₁₋₆)-carbonyl, Carbamoyl, N-Alkyl(C₁₋₆₎carbamoyl, N,N-Dialkyl(C₁₋₆)-carbamoyl, Alkyl(C₁₋₆)sulfonyl, Formyl, Trifluoralkyl(C₁₋₆)-carbonyl, Alkyl(C₁₋₆)carboxy oder Thiocarbamoyl ausgewählt ist;
• die Gruppen R₅ und R₆, die gleich oder verschieden sein können, ein Wasserstoffatom; ein Halogenatom; Alkyl(C₁₋₆)carbonyl; Aminoalkyl(C₁₋₆)carbonyl; N-Alkyl(C₁₋₆)aminoalkyl-(C₁₋₆)carbonyl; N,N-Dialkyl(C₁₋₆)aminoalkyl(C₁₋₆)carbonyl; Aminoalkyl(C₁₋₆-)carbonylalkyl(C₁₋₆); N-Alkyl(C₁₋₆)aminoalkyl-(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Alkyl(C₁₋₆)aminosulfonyl; N,N-Dialkyl(C₁₋₆)-aminosulfonyl; Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Alkyl(C₁₋₆); Monohydroxyalkyl(C₁₋₆); Polyhydroxyalkyl(C₂₋₆); Alkoxy(C₁₋₆)alkyl(C₁₋₆); Trifluoralkyl(C₁₋₆); Aminoalkyl(C₁₋₆); wobei die Alkylgruppe unsubstituiert vorliegt oder mit einer oder mehreren Hydroxygruppen substituiert ist und wobei die Aminogruppe unsubstituiert vorliegt oder mit einer oder zwei Gruppen substituiert ist, die gleich oder verschieden sind und unter den folgenden Gruppen ausgewählt sind: Alkyl(C₁₋₆), Monohydroxyalkyl(C₁₋₆), Polyhydroxyalkyl(C₂₋₆), Alkyl(C₁₋₆)carbonyl, Carbamoyl, N-Alkyl(C₁₋₆)carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl; oder eine nachfolgend definierte Gruppe OR₇ oder -SR₇;
• R₇ ein Wasserstoffatom; Alkyl(C₁₋₆); Monohydroxyalkyl(C₁₋₆); Polyhydroxyalkyl(C₂₋₆); Alkoxy(C₁₋₆)alkyl(C₁₋₆); Aryl; Benzyl; Carboxyalkyl(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Trifluoralkyl(C₁₋₆); Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); Aminoalkyl(C₁₋₆), wobei die Alkylgruppe unsubstituiert oder mit einer oder mehreren Hydroxygruppen substituiert ist; Aminoalkyl(C₁₋₆), wobei die Alkylgruppe unsubstituiert oder mit einer oder mehreren Hydroxygruppen substituiert ist und wobei die Aminogruppe mit einer oder zwei Gruppen substituiert ist, die gleich oder verschieden sind und unter den Gruppen Alkyl(C₁₋₆), Monohydroxyalkyl(C₁₋₆), Polyhydroxyalkyl(C₂₋₆), Alkyl(C₁₋₆)carbonyl, Formyl, Trifluoralkyl(C₁₋₆)carbonyl, Alkyl-(C₁₋₆)carboxy, Carbamoyl, N-Alkyl(C₁₋₆)carbamoyl, N,N-Dialkyl-(C₁₋₆)carbamoyl, Thiocarbamoyl, Alkyl(C₁₋₆)sulfonyl ausgewählt sind;
• Z ist unter den ungesättigten kationischen Gruppen der Formel (II) und den gesättigten kationischen Gruppen der Formel (III) ausgewählt: 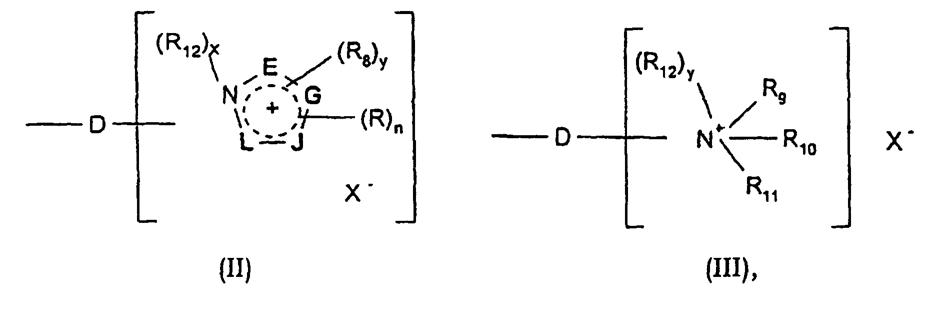
worin bedeuten:
• D eine Verbindungsgruppe, die eine geradkettige oder verzweigte Alkylgruppe ist, die vorzugsweise 1 bis 14 Kohlenstoffatome aufweist und die mit einer oder mehreren Hydroxygruppen oder C₁₋₆-Alkoxygruppen substituiert sein kann;
• die Ringbestandteile E, G, J und L, die gleich oder verschieden sind, ein Kohlenstoffatom oder ein Stickstoffatom;
• n 0 oder eine ganze Zahl von 1 bis 4;
• die Gruppen R, die gleich oder verschieden sind, ein Halogenatom, eine Hydroxygruppe, Alkyl(C₁₋₆), Monohydroxyalkyl(C₁₋₆), Polyhydroxyalkyl(C₂₋₆), Nitro, Cyano, Cyanoalkyl(C₁₋₆), Alkoxy(C₁₋₆), Trialkyl(C₁₋₆)silanalkyl(C₁₋₆), Amido, Aldehydo, Carboxy, Alkylcarbonyl(C₁₋₆), Thio, Thioalkyl(C₁₋₆), Alkyl(C₁₋₆)thio, Amino, Amino, wobei die Aminogruppe mit Alkyl(C₁₋₆)carbonyl, Carbamoyl oder Alkyl(C₁₋₆)sulfonyl geschützt ist: NHR" oder NR"R"' wobei die Gruppen R" und R"' die gleich oder verschieden sind, Alkyl(C₁₋₆), Monohydroxyalkyl(C₁₋₆) oder Polyhydroxyalkyl(C₂₋₆) bedeuten;
• R₈ Alkyl(C₁₋₆), Monohydroxyalkyl(C₁₋₆), Polyhydroxyalkyl(C₂₋₆), Cyanoalkyl(C₁₋₆), Trialkyl(C₁₋₆)silanalkyl(C₁₋₆), Alkoxy(C₁₋₆)alkyl-(C₁₋₆), Carbamoylalkyl(C₁₋₆), Alkyl(C₁₋₆)carboxyalkyl(C₁₋₆) oder Benzyl;
• die Gruppen R₉, R₁₀ und R₁₁, die gleich oder verschieden sind, Alkyl(C₁₋₆), Monohydroxyalkyl(C₁₋₆), Polyhydroxyalkyl(C₂₋₆), Alkyloxy(C₁₋₆)alkyl(C₁₋₆), Cyanoalkyl(C₁₋₆), Aryl, Benzyl, Amidoalkyl(C₁₋₆), Trialkyl(C₁₋₆)silanalkyl(C₁₋₆) oder Aminoalkyl(C₁₋₆), wobei die Aminogruppe mit Alkyl(C₁₋₆)carbonyl, Carbamoyl oder Alkyl(C₁₋₆)sulfonyl geschützt ist; wobei zwei der Gruppen R₉, R₁₀ und R₁₁ auch gemeinsam mit dem Stickstoffatom, an das sie gebunden sind, einen gesättigten, 5- oder 6-gliedrigen Ring auf Kohlenstoffbasis oder Ring, der ein oder mehrere Heteroatome enthalten kann, wie beispielsweise Pyrrolidin, Piperidin, Piperazin oder Morpholin, bilden können, wobei der Ring unsubstituiert vorliegt oder mit einem Halogenatom, Hydroxy, Alkyl(C₁₋₆), Monohydroxyalkyl(C₁₋₆), Polyhydroxyalkyl(₂₋₆), Nitro, Cyano, Cyanoalkyl(C₁₋₆), Alkoxy(C₁₋₆), Trialkyl(C₁₋₆)silanalkyl(C₁₋₆), Amido, Aldehydo, Carboxy, Ketoalkyl(C₁₋₆), Thio, Thioalkyl(C₁₋₆), Alkyl(C₁₋₆)thio, Amino und Aminogruppen, die mit Alkyl(C₁₋₆)carbonyl, Carbamoyl oder Alkyl(C₁₋₆)sulfonyl geschützt sind, substituiert sein kann;
• R₁₂ Alkyl(C₁₋₆); Monohydroxyalkyl(C₁₋₆); Polyhydroxyalkyl(C₂₋₆); Aryl; Benzyl; Aminoalkyl(C₁₋₆), Aminoalkyl(C₁₋₆), wobei die Aminogruppe mit Alkyl(C₁₋₆)carbonyl, Carbamoyl oder Alkyl(C₁₋₆)-sulfonyl geschützt ist; Carboxyalkyl(C₁₋₆); Cyanoalkyl(C₁₋₆); Carbamoylalkyl(C₁₋₆); Trifluoralkyl(C₁₋₆); Trialkyl(C₁₋₆)silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
• x und y bedeuten 0 oder 1; mit den folgenden Maßgaben:
- in den ungesättigten kationischen Gruppen der Formel (II):
- wenn x = 0, ist die Verbindungsgruppe D an das Stickstoffatom gebunden,
- wenn x = 1, ist die Verbindungsgruppe D an einen Ringbestandteil E, G, J oder L gebunden,
- y kann nur unter den folgenden Bedingungen den Wert 1 annehmen:
1) wenn die Ringbestandteile E, G, J und L gleichzeitig ein Kohlenstoffatom bedeuten und sich die Gruppe R₈ an dem Stickstoffatom des ungesättigten Rings befindet; oder
2) wenn mindestens ein Ringbestandteil E, G, J oder L ein Stickstoffatom bedeutet, an das die Gruppe R₈ gebunden ist;
- in den kationischen Gruppen der Formel (III):
- wenn y = 0, ist die Verbindungsgruppe D an das Stickstoffatom gebunden, das die Gruppen R₉ bis R₁₁ trägt,
- wenn y = 1, bilden zwei der Gruppen R₉ bis R₁₁ gemeinsam mit dem Stickstoffatom, an das sie gebunden sind, einen 5- oder 6-gliedrigen gesättigten Ring, wie er oben definiert wurde, und die Verbindungsgruppe D befindet sich an einem Kohlenstoffatom dieses gesättigten Rings;
• X⁻ bedeutet ein einwertiges oder zweiwertiges Anion; mit der Maßgabe, dass die Anzahl der ungesättigten kationischen Gruppen Z der Formel (II), bei denen mindestens einer der Ringbestandteile E, G, J und L ein Stickstoffatom bedeutet, mindestens 1 beträgt.

2. Kationische Nitro-phenylendiamine auf Monobenzolbasis nach Anspruch 1, **dadurch gekennzeichnet, dass** die Ringe der ungesättigten Gruppen Z der Formel (II) unter Pyrrol, Imidazol, Pyrazol und Triazol ausgewählt sind.

3. Kationische Nitro-phenylendiamine auf Monobenzolbasis nach einem der vorhergehenden Ansprüche, **dadurch gekennzeichnet, dass** in der Formel (III) zwei der Gruppen R₉, R₁₀ und R₁₁ einen Pyrrolidinring, Piperdinring, Piperazinring oder Morpholinring bilden, wobei der Ring gegebenenfalls mit einem Halogenatom, einer Hydroxygruppe, C₁₋₆-Alkyl, C₁₋₆-Mohohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, Alkylcarbonyl(C₁₋₆), Thio, Thioalkyl(C₁₋₆), Alkyl(C₁₋₆)thio, Amino oder einer Aminogruppe, die mit Alkyl(C₁₋₆)carbonyl, Carbamoyl oder Alkyl(C₁₋₆)sulfonyl geschützt ist, substituiert sein kann.

4. Kationische Nitro-phenylendiamine auf Monobenzolbasis nach einem der vorhergehenden Ansprüche, **dadurch gekennzeichnet, dass** X⁻ unter den Halogenatomen, Hydroxid, Hydrogensulfat oder Alkyl(C₁₋₆)sulfat ausgewählt ist.

5. Kationische Nitro-phenylendiamine auf Monobenzolbasis nach einem der vorhergehenden Ansprüche, **dadurch gekennzeichnet, dass** sie unter den Verbindungen der folgenden Formeln (I)₁ bis (I)₁₅ ausgewählt sind:

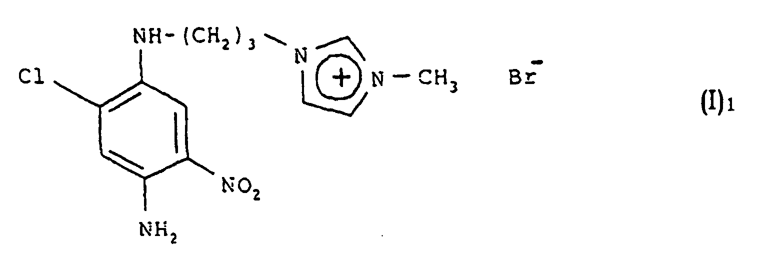

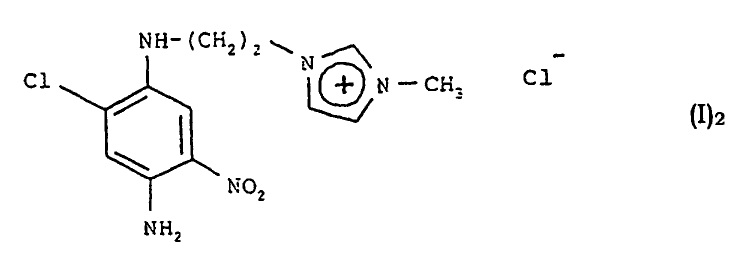

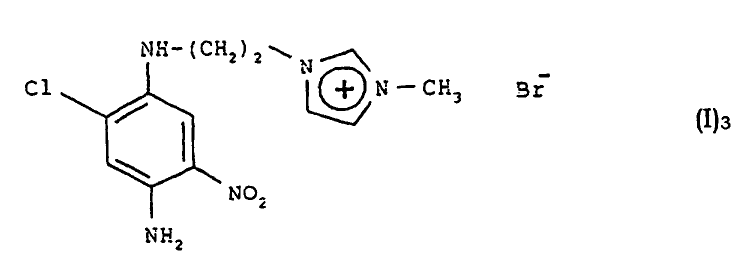

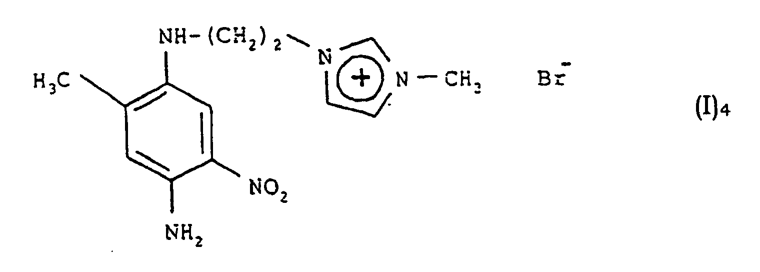

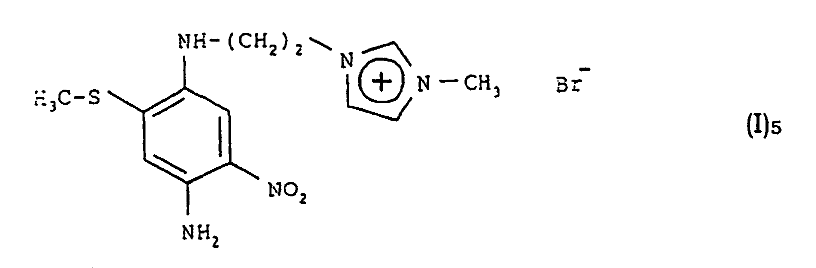

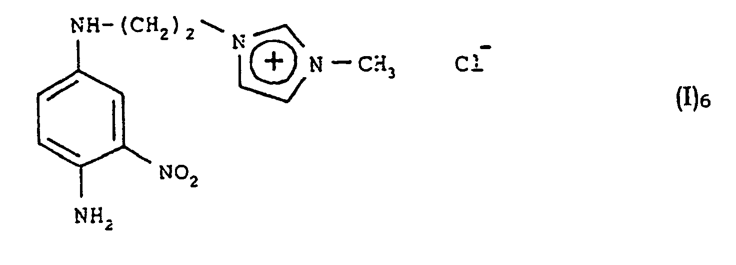

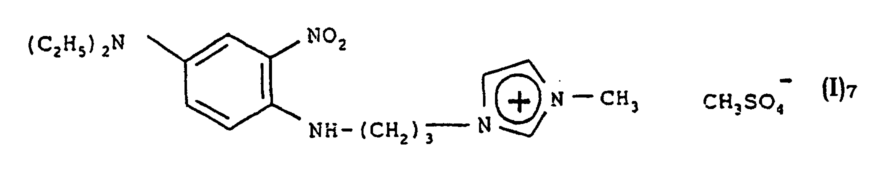

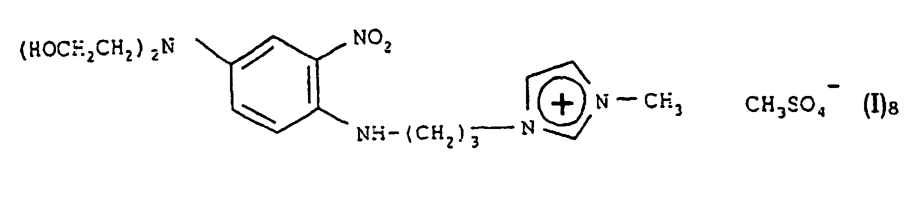

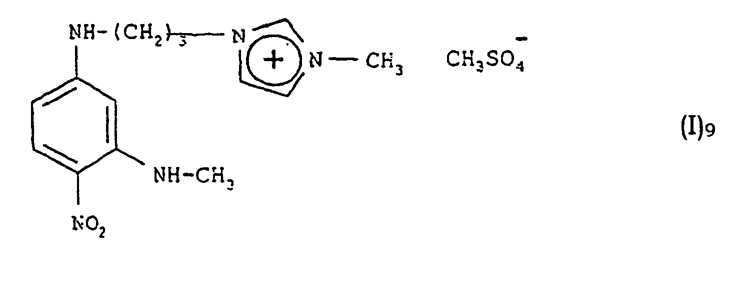

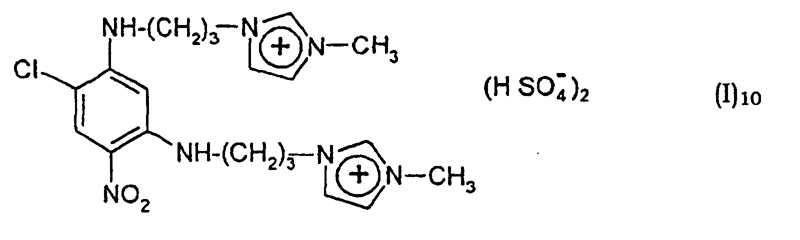

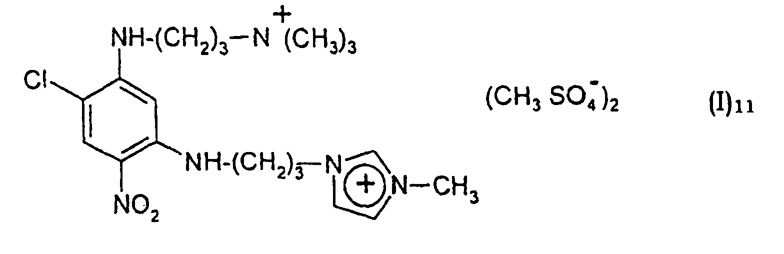

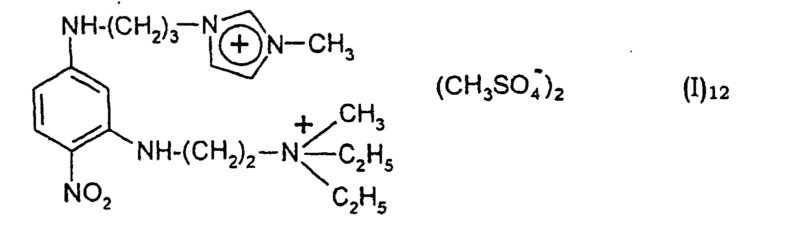

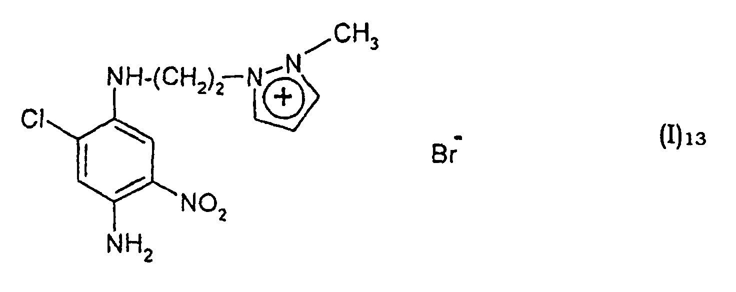

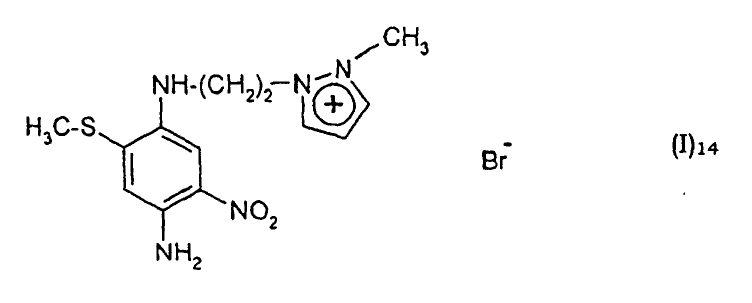

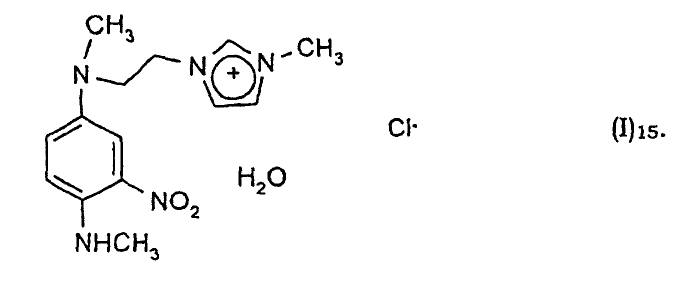

6. Kationische Nitro-phenylendiamine auf Monobenzolbasis nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

7. Verwendung von kationischen Nitro-phenylendiaminen auf Monobenzolbasis der Formel (I) nach einem der Ansprüche 1 bis 6 als Direktfarbstoffe in Zusammensetzungen zum Färben von Keratinfasern und insbesondere menschlichen Keratinfasern und besonders Haaren oder zur Herstellung solcher Zusammensetzungen.

8. Zusammensetzung zum Färben von Keratinsubstanzen, **dadurch gekennzeichnet, dass** sie in einem zum Färben geeigneten Medium eine wirksame Menge mindestens eines kationischen Nitro-phenylendiamin auf Monobenzolbasis der Formel (I) nach einem der Ansprüche 1 bis 6 enthält.

9. Zusammensetzung zum direkten Färben von menschlichen Keratinfasern und insbesondere Haaren, **dadurch gekennzeichnet, dass** sie in einem zum Färben geeigneten Medium eine wirksame Menge mindestens eines kationischen Nitro-phenylendiamin auf Monobenzolbasis der Formel (I) nach einem der Ansprüche 1 bis 6 enthält.

10. Zusammensetzung nach den Ansprüchen 8 oder 9, **dadurch gekennzeichnet, dass** sie einen pH-Wert im Bereich von 3 bis 12 besitzt.

11. Zusammensetzung nach einem der Ansprüche 8 bis 10, **dadurch gekennzeichnet, dass** die kationischen Nitro-phenylendiamine auf Monobenzolbasis der Formel (I) in einer Konzentration von 0,005 bis 12 Gew.-%, bezogen auf das Gesamtgewicht der Zusammensetzung, enthalten sind.

12. Zusammensetzung nach Anspruch 11, **dadurch gekennzeichnet, dass** die kationische Nitro-phenylendiamine auf Monobenzolbasis der Formel (I) in einer Konzentration von 0,05 bis 6 Gew.-%, bezogen auf das Gesamtgewicht der Zusammensetzung, enthalten sind.

13. Zusammensetzung nach einem der Ansprüche 8 bis 12, **dadurch gekennzeichnet, dass** sie weitere Direktfarbstoffe enthält, die unter den nitrierten Benzolfarbstoffen, die von den erfindungsgemäßen kationischen Nitro-phenylendiaminen der Formel (I) verschieden sind, wie Nitrodiphenylaminen, Nitroanilinen, nitrierten Phenolethern oder Nitrophenolen, Nitropyridinen, Anthrachinon-Farbstoffen, Mono- oder Diazofarbstoffen, Triarylmethan-Farbstoffen, Azin-Farbstoffen, Acridin-Farbstoffen, Xanthen-Farbstoffen oder metallhaltigen Farbstoffen ausgewählt sind.

14. Zusammensetzung nach einem der Ansprüche 8 bis 13, **dadurch gekennzeichnet, dass** das zum Färben geeignete Medium ein wässriges Medium ist, das aus Wasser und/oder organischen Lösungsmitteln, die unter den Alkoholen, Glykolen und Glykolethern ausgewählt sind, in Mengenanteilen von 1 bis 40 Gew.-%, bezogen auf das Gesamtgewicht der Zusammensetzung, besteht.

15. Verfahren zum Färben von Keratinfasern und insbesondere menschlichen Keratinfasern, wie Haaren, durch direkte Färbung, **dadurch gekennzeichnet, dass** die Farbmittelzusammensetzung nach einem der Ansprüche 8 bis 14 auf die trockenen oder feuchten Keratinfasern aufgetragen wird und die Fasern ohne zwischenzeitliches Spülen getrocknet werden.

16. Verfahren zum Färben von Keratinfasern und insbesondere menschlichen Keratinfasern, wie Haaren, durch direkte Färbung, **dadurch gekennzeichnet, dass** die Farbmittelzusammensetzung nach einem der Ansprüche 8 bis 14 auf die trockenen oder feuchten Fasern aufgetragen wird und die Fasern, nachdem die Zusammensetzung etwa 3 bis 60 Minuten einwirken gelassen wurde, gespült, gegebenenfalls gewaschen, nochmals gespült und dann getrocknet werden.

## Claims

1. Cationic monobenzene nitrophenylenediamines of formula (I) and their addition salts with an acid: 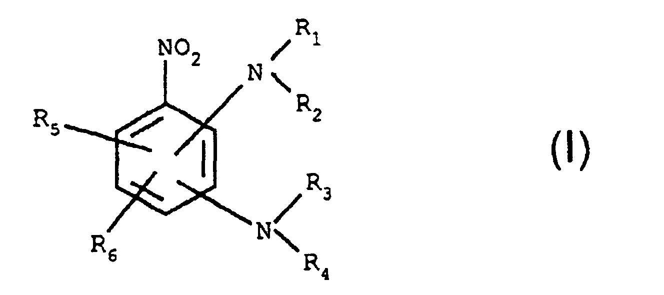 in which formula,
• **R**_{**1**}, **R**_{**2**}, **R**_{**3**} and **R**_{**4**}, which can be identical or different, represent a hydrogen atom; a Z group defined below; a (C₁-C₆)alkyl radical; a monohydroxy(C₁-C₆)alkyl radical; a polyhydroxy(C₂-C₆)alkyl radical; a (C₁-C₆)alkoxy(C₁-C₆)alkyl radical; an aryl radical; a benzyl radical; an N,N-di(C₁-C₆)alkylcarbamyl (C₁-C₆)alkyl radical; a thiocarbamyl(C₁-C₆)alkyl radical; a trifluoro(C₁-C₆)alkyl radical; a sulpho(C₁-C₆)alkyl radical; a (C₁-C₆)alkylcarboxy(C₁-C₆)alkyl radical; a (C₁-C₆)alkylsulphinyl(C₁-C₆)alkyl radical; an aminosulphonyl(C₁-C₆)alkyl radical; an N-(C₁-C₆)alkylaminosulphonyl(C₁-C₆)alkyl radical; an N,N-di(C₁-C₆)alkylaminosulphonyl(C₁-C₆)alkyl radical; a (C₁-C₆)alkylcarbonyl (C₁-C₆)alkyl radical; an amino(C₁-C₆)alkyl radical, the alkyl of which is substituted or unsubstituted by one or more hydroxyl radicals; or an amino(C₁-C₆)alkyl radical, the alkyl of which is substituted or unsubstituted by one or more hydroxyl radicals and the amine of which is substituted by one or two identical or different radicals chosen from alkyl, monohydroxy(C₁-C₆)alkyl, polyhydroxy(C₂-C₆) alkyl, (C₁-C₆)alkylcarbonyl, carbamyl, N-(C₁-C₆)alkylcarbamyl or N,N-di(C₁-C₆)alkylcarbamyl, (C₁-C₆)alkylsulphonyl, formyl, trifluoro(C₁-C₆)alkylcarbonyl, (C₁-C₆)alkylcarboxyl or thiocarbamyl radicals;
• **R**_{**5**} and **R**_{**6**}, which can be identical or different, represent a hydrogen atom; a halogen atom; a (C₁-C₆) alkylcarbonyl radical; an amino (C₁-C₆) alkylcarbonyl radical; an N-(C₁-C₆)alkylamino(C₁-C₆)alkylcarbonyl radical; an N,N-di(C₁-C₆)alkylamino(C₁-C₆)alkylcarbonyl radical; an amino(C₁-C₆)alkylcarbonyl (C₁-C₆)alkyl radical; an N-(C₁-C₆)alkylamino (C₁-C₆)alkylcarbonyl (C₁-C₆)alkyl radical; an N,N-di(C₁-C₆)alkylamino (C₁-C₆)alkylcarbonyl(C₁-C₆)alkyl radical; a carboxyl radical; a (C₁-C₆)alkylcarboxyl radical; a (C₁-C₆)alkylsulphonyl radical; an aminosulphonyl radical; an N-(C₁-C₆)alkylaminosulphonyl radical; an N,N-di(C₁-C₆)alkylaminosulphonyl radical; an aminosulphonyl(C₁-C₆)alkyl radical; an N-(C₁-C₆)alkylaminosulphonyl(C₁-C₆)alkyl radical; an N,N-di(C₁-C₆)alkylaminosulphonyl(C₁-C₆)alkyl radical; a carbamyl radical; an N-(C₁-C₆)alkylcarbamyl radical; an N,N-di(C₁-C₆)alkylcarbamyl radical; a carbamyl (C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a (C₁-C₆)alkyl radical; a monohydroxy(C₁-C₆)alkyl radical; a polyhydroxy(C₂-C₆)alkyl radical; a (C₁-C₆) alkoxy (C₁-C₆) alkyl radical; a trifluoro(C₁-C₆)alkyl radical; an amino(C₁-C₆)alkyl radical, the alkyl of which is substituted or unsubstituted by one or more hydroxyl radicals and the amine of which is substituted or unsubstituted by one or two identical or different radicals chosen from (C₁-C₆)alkyl, monohydroxy(C₁-C₆)alkyl, polyhydroxy(C₂-C₆)alkyl, (C₁-C₆)alkylcarbonyl, carbamyl, N-(C₁-C₆)alkylcarbamyl or N, N-di (C₁-C₆)alkylcarbamyl, (C₁-C₆)alkylsulphonyl, formyl, trifluoro(C₁-C₆)alkylcarbonyl, (C₁-C₆)alkylcarboxyl or thiocarbamyl radicals; or an OR₇ or -SR₇ group defined below;
• **R**_{**7**} denotes a hydrogen atom; a (C₁-C₆)alkyl radical; a monohydroxy(C₁-C₆)alkyl radical; a polyhydroxy (C₂-C₆)alkyl radical; a (C₁-C₆)alkoxy(C₁-C₆)alkyl radical; an aryl radical; a benzyl radical; a carboxy(C₁-C₆)alkyl radical; a (C₁-C₆)alkylcarboxy(C₁-C₆)alkyl radical; a cyano (C₁-C₆)alkyl radical; a carbamyl (C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a trifluoro(C₁-C₆)alkyl radical; an aminosulphonyl(C₁-C₆)alkyl radical; an N-(C₁-C₆)alkylaminosulphonyl(C₁-C₆)alkyl radical; an N,N-di(C₁-C₆)alkylaminosulphonyl (C₁-C₆) alkyl radical; a (C₁-C₆) alkylsulphinyl(C₁-C₆)alkyl radical; a (C₁-C₆)alkylsulphonyl(C₁-C₆)alkyl radical; a (C₁-C₆)alkylcarbonyl (C₁-C₆)alkyl radical; an amino (C₁-C₆)alkyl radical, the alkyl of which is substituted or unsubstituted by one or more hydroxyl radicals; or an amino (C₁-C₆) alkyl radical, the alkyl of which is substituted or unsubstituted by one or more hydroxyl radicals and the amine of which is substituted by one or two identical or different radicals chosen from (C₁-C₆)alkyl, monohydroxy(C₁-C₆)alkyl, polyhydroxy(C₂-C₆)alkyl, (C₁-C₆)alkylcarbonyl, formyl, trifluoro (C₁-C₆)alkylcarbonyl, (C₁-C₆)alkylcarboxyl, carbamyl, N-(C₁-C₆)alkylcarbamyl, N,N-di(C₁-C₆)alkylcarbamyl, thiocarbamyl or (C₁-C₆)alkylsulphonyl radicals;
• Z is chosen from the unsaturated cationic groups of following formula (II) and the saturated cationic groups of following formula (III): 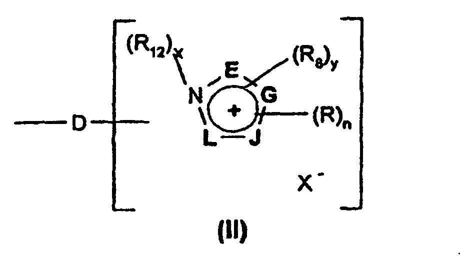 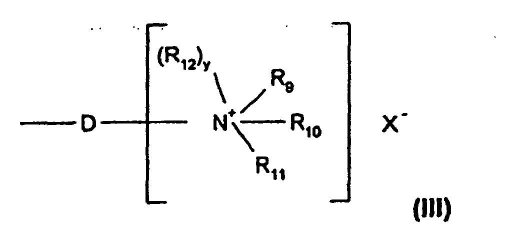
in which formulae:
• D is a linking arm which represents a linear or branched alkyl chain preferably comprising from 1 to 14 carbon atoms which can be substituted by one or more hydroxyl or (C₁-C₆)alkoxy radicals;
• the E, G, J and L vertices, which are identical or different, represent a carbon or nitrogen atom;
• n is an integer between 0 and 4 inclusive;
• the R radicals, which are identical or different, represent a halogen atom, a hydroxyl radical, a (C₁-C₆)alkyl radical, a monohydroxy(C₁-C₆)alkyl radical, a polyhydroxy(C₂-C₆)alkyl radical, a nitro radical, a cyano radical, a cyano(C₁-C₆)alkyl radical, a (C₁-C₆)alkoxy radical, a tri(C₁-C₆)alkylsilyl(C₁-C₆)alkyl radical, an amido radical, a aldehydo radical, a carboxyl radical, a (C₁-C₆)alkylcarbonyl radical, a thio radical, a thio(C₁-C₆)alkyl radical, a (C₁-C₆)alkylthio radical, an amino radical, an amino radical protected by a (C₁-C₆)alkylcarbonyl, carbamyl or (C₁-C₆)alkylsulphonyl radical, or an NHR" or NR"R"' group in which R" and R"', which are identical or different, represent a (C₁-C₆)alkyl radical, a monohydroxy(C₁-C₆)alkyl radical or a polyhydroxy(C₂-C₆)alkyl radical;
• R₈ represents a (C₁-C₆)alkyl radical, a monohydroxy(C₁-C₆)alkyl radical, a polyhydroxy(C₂-C₆)alkyl radical, a cyano(C₁-C₆)alkyl radical, a tri(C₁-C₆)alkylsilyl(C₁-C₆)alkyl radical, a (C₁-C₆)alkoxy(C₁-C₆)alkyl radical, a carbamyl (C₁-C₆) alkyl radical, a (C₁-C₆)alkylcarboxy(C₁-C₆)alkyl radical or a benzyl radical;
• R₉, R₁₀ and R₁₁, which are identical or different, represent a (C₁-C₆)alkyl radical, a monohydroxy(C₁-C₆)alkyl radical, a polyhydroxy(C₂-C₆)alkyl radical, a (C₁-C₆)alkoxy-(C₁-C₆)alkyl radical, a cyano(C₁-C₆)alkyl radical, an aryl radical, a benzyl radical, an amido(C₁-C₆)alkyl radical, a tri(C₁-C₆)alkylsilyl(C₁-C₆)alkyl radical or an amino (C₁-C₆)alkyl radical, the amine of which is protected by a (C₁-C₆)alkylcarbonyl, carbamyl or (C₁-C₆)alkylsulphonyl radical; two of the R₉, R₁₀ and R₁₁ radicals can also together form, with the nitrogen atom to which they are attached, a saturated carbonaceous 5- or 6-membered ring or a saturated 5- or 6-membered ring which can comprise one or more heteroatoms, such as, for example, a pyrrolidine ring, a piperidine ring, a piperazine ring or a morpholine ring, it being possible for the said ring to be substituted or unsubstituted by a halogen atom, a hydroxyl radical, a (C₁-C₆)alkyl radical, a monohydroxy(C₁-C₆)alkyl radical, a polyhydroxy(C₂-C₆)alkyl radical, a nitro radical, a cyano radical, a cyano(C₁-C₆)alkyl radical, a (C₁-C₆)alkoxy radical, a tri(C₁-C₆)alkylsilyl(C₁-C₆)alkyl radical, an amido radical, a formyl radical, a carboxyl radical, a keto(C₁-C₆)alkyl radical, a thio radical, a thio(C₁-C₆)alkyl radical, a (C₁-C₆)alkylthio radical, an amino radical or an amino radical protected by a (C₁-C₆)alkylcarbonyl, carbamyl or (C₁-C₆)alkylsulphonyl radical;
• R₁₂ represents a (C₁-C₆)alkyl radical; a monohydroxy(C₁-C₆)alkyl radical; a polyhydroxy(C₂-C₆)alkyl radical; an aryl radical; a benzyl radical; an amino (C₁-C₆)alkyl radical; an amino(C₁-C₆)alkyl radical, the amine of which is protected by a (C₁-C₆)alkylcarbonyl, carbamyl or (C₁-C₆)alkylsulphonyl radical; a carboxy(C₁-C₆)alkyl radical; a cyano(C₁-C₆)alkyl radical; a carbamyl-(C₁-C₆)alkyl radical; a trifluoro(C₁-C₆)alkyl radical; a tri(C₁-C₆)alkylsilyl(C₁-C₆)alkyl radical; a sulphonamido(C₁-C₆)alkyl radical; a (C₁-C₆)alkylcarboxy(C₁-C₆)alkyl radical; a (C₁-C₆)alkylsulphinyl (C₁-C₆) alkyl radical; a (C₁-C₆)alkylsulphonyl(C₁-C₆)alkyl radical; a (C₁-C₆)alkylketo(C₁-C₆)alkyl radical; an N-(C₁-C₆)alkylcarbamyl(C₁-C₆)alkyl radical; or an N-(C₁-C₆)alkylsulphonamido(C₁-C₆)alkyl radical;
• x and y are integers equal to 0 or 1; with the following conditions:
- in the unsaturated cationic groups of formula (II):
- when x = 0, the D linking arm is attached to the nitrogen atom,
- when x = 1, the D linking arm is attached to one of the E, G, J or L vertices,
- y can take the value 1 only:
1) when the E, G, J and L vertices simultaneously represent a carbon atom and when the R₈ radical is carried by the nitrogen atom of the unsaturated ring; or else
2) when at least one of the E, G, J and L vertices represents a nitrogen atom to which the R₈ radical is attached;
- in the cationic groups of formula (III):
- when y = 0, then the D linking arm is attached to the nitrogen atom carrying the R₉ to R₁₁ radicals,
- when y = 1, then two of the R₉ to R₁₁ radicals form, jointly with the nitrogen atom to which they are attached, a saturated 5- or 6-membered ring as defined above and the D linking arm is carried by a carbon atom of the said saturated ring;
• **X**⁻ represents a monovalent or divalent anion,
**it being understood** that the number of Z unsaturated cationic groups of formula (II) in which at least one of the E, G, J and L vertices represents a nitrogen atom is at least equal to 1.

2. Cationic monobenzene nitrophenylenediamines according to Claim 1, **characterized in that** the rings of the Z unsaturated groups of formula (II) are chosen from the pyrrole, imidazole, pyrazole and triazole rings.

3. Cationic monobenzene nitrophenylenediamines according to either one of the preceding claims, **characterized in that**, in the formula (III), two of the R₉, R₁₀ and R₁₁ radicals form a pyrrolidine ring, a piperidine ring, a piperazine ring or a morpholine ring, it being possible for the said ring to be substituted or unsubstituted by a halogen atom, a hydroxyl radical, a (C₁-C₆)alkyl radical, a monohydroxy(C₁-C₆)alkyl radical, a polyhydroxy(C₂-C₆)alkyl radical, a nitro radical, a cyano radical, a cyano(C₁-C₆)alkyl radical, a (C₁-C₆)alkoxy radical, a tri (C₁-C₆)alkylsilyl(C₁-C₆)alkyl radical, an amido radical, a formyl radical, a carboxyl radical, a (C₁-C₆)alkylcarbonyl radical, a thio radical, a thio(C₁-C₆)alkyl radical, a (C₁-C₆)alkylthio radical, an amino radical or an amino radical protected by a (C₁-C₆)alkylcarbonyl, carbamyl or (C₁-C₆)alkylsulphonyl radical.

4. Cationic monobenzene nitrophenylenediamines according to any one of the preceding claims, **characterized in that** X⁻ is chosen from a halogen atom, a hydroxide, a hydrogen sulphate or a (C₁-C₆)alkyl sulphate.

5. Cationic monobenzene nitrophenylenediamines according to any one of the preceding claims, **characterized in that** they are chosen from those of following formulae (I)₁ to (I)₁₅:

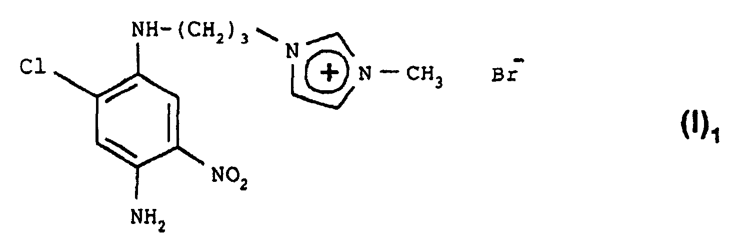

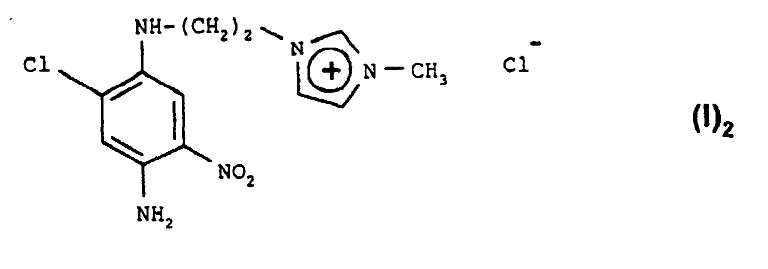

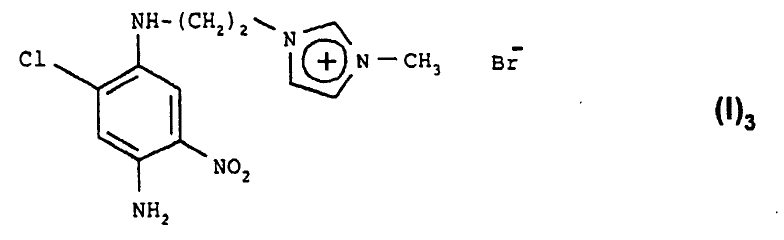

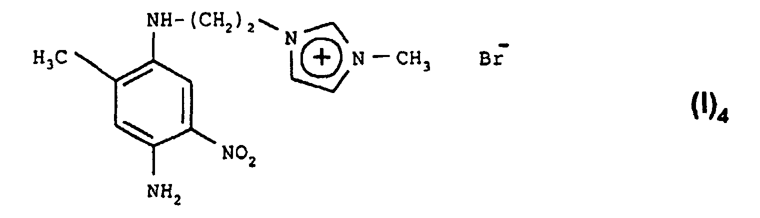

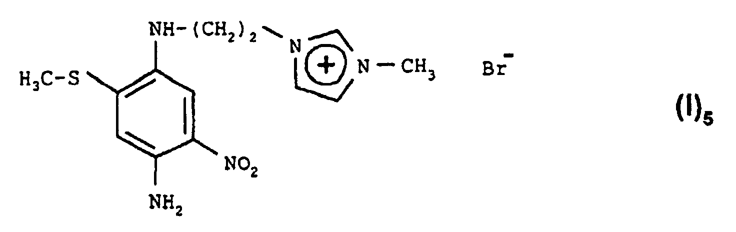

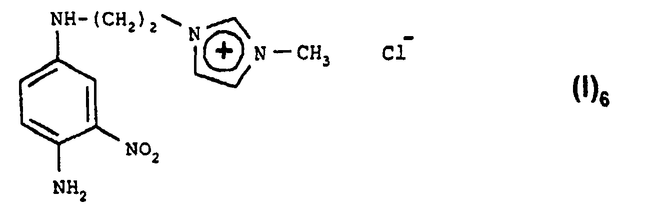

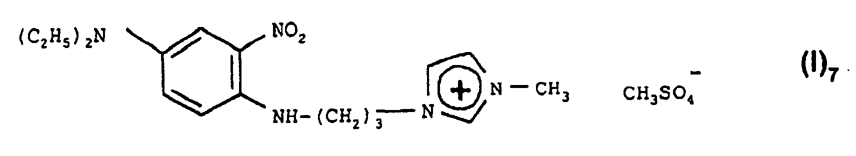

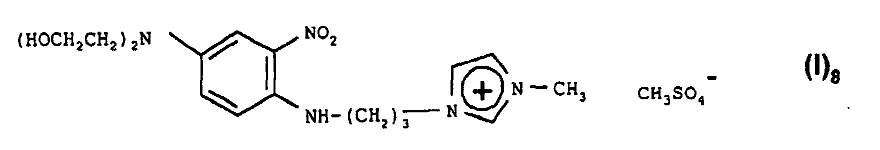

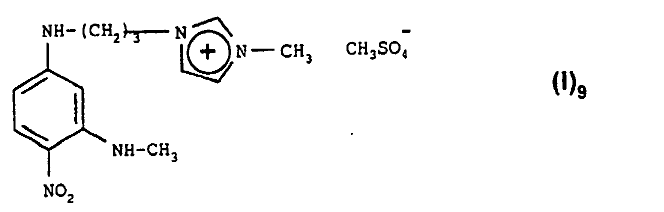

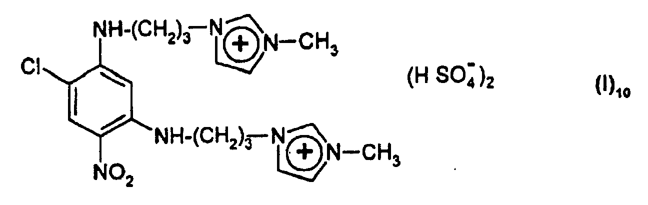

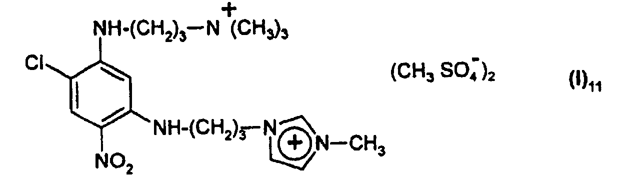

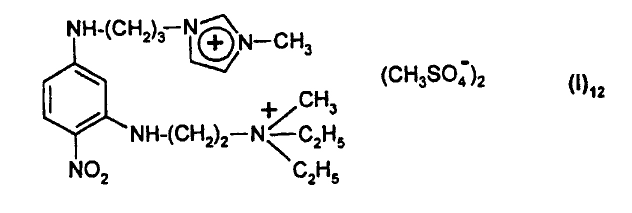

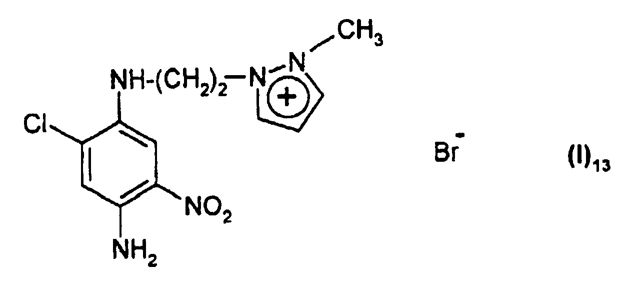

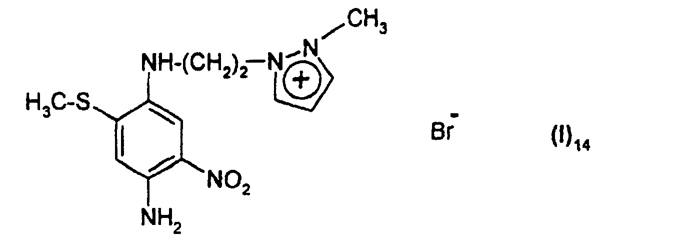

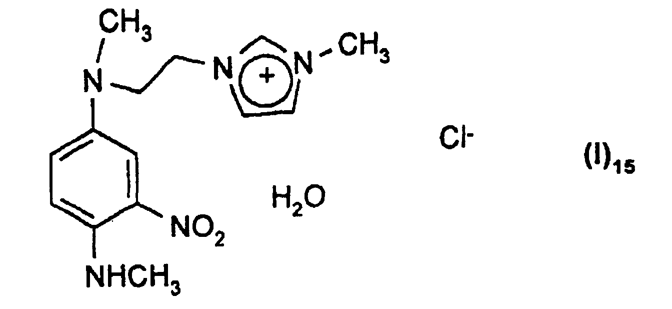

6. Cationic monobenzene nitrophenylenediamines according to any one of the preceding claims, **characterized in that** the addition salts with an acid are chosen from hydrochlorides, hydrobromides, sulphates, citrates, succinates, tartrates, lactates and acetates.

7. Use of the cationic monobenzene nitrophenylenediamines of formula (I) as defined in any one of Claims 1 to 6 as direct dyes in or for the manufacture of dyeing compositions for keratinous substances, in particular for human keratinous fibres and especially the hair.

8. Dyeing composition for keratinous substances, **characterized in that** it comprises, in a medium appropriate for dyeing, an effective amount of at least one cationic monobenzene nitrophenylenediamine of formula (I) defined in any one of Claims 1 to 6.

9. Direct dyeing composition for human keratinous fibres and especially the hair, **characterized in that** it comprises, in a medium appropriate for dyeing, an effective amount of at least one cationic monobenzene nitrophenylenediamine of formula (I) defined in any one of Claims 1 to 6.

10. Composition according to Claims 8 and 9, **characterized in that** it has a pH of between 3 and 12.

11. Composition according to any one of Claims 8 to 10, **characterized in that** the cationic monobenzene nitrophenylenediamines of formula (I) are present in a concentration ranging from 0.005 to 12% by weight with respect to the total weight of the composition.

12. Composition according to Claim 11, **characterized in that** the cationic monobenzene nitrophenylenediamines of formula (I) are present in a concentration ranging from 0.05 to 6% by weight with respect to the total weight of the composition.

13. Composition according to any one of Claims 8 to 12, **characterized in that** it comprises other direct dyes chosen from nitrobenzene dyes other than the cationic nitrophenylenediamines of formula (I) according to the invention and such as nitrodiphenylamines, nitroanilines, nitrophenol ethers or nitrophenols, nitropyridines, anthraquinone dyes, mono- or diazo, triarylmethane, azine, acridine or xanthene dyes or metalliferous dyes.

14. Composition according to any one of Claims 8 to 13, **characterized in that** the medium appropriate for dyeing is an aqueous medium composed of water and/or of organic solvents chosen from alcohols, glycols and glycol ethers in proportions of between 1 and 40% by weight with respect to the total weight of the composition.

15. Process for dyeing keratinous fibres and in particular human keratinous fibres, such as the hair, by direct colouring, **characterized in that** the dyeing composition defined in any one of Claims 8 to 14 is applied to dry or wet keratinous fibres and **in that** these fibres are dried without intermediate rinsing.

16. Process for dyeing keratinous fibres and in particular human keratinous fibres, such as the hair, by direct colouring, **characterized in that** the dyeing composition defined in any one of Claims 8 to 14 is applied to dry or wet keratinous fibres and **in that**, after having allowed the composition to act for 3 to 60 minutes approximately, the fibres are rinsed, are optionally washed, are again rinsed and are then dried.
